# EUROPEAN PATENT APPLICATION

(11) **EP 4 130 288 A1**
(43) Date of publication of application: **08.02.2023**
(21) Application number: 21782016.6
(22) Date of filing: 01.04.2021
(51) Int. Cl.: C12Q 1/28, G01N 1/28, G01N 1/30, G01N 1/36, G01N 33/48, G01N 33/536, G01N 33/58

(54) **STAINING METHOD, MICROSCOPIC OBSERVATION METHOD, STAINING AGENT AND STAINING KIT**

(30) Priority: 02.04.2020 JP 2020066711
(71) Applicant: Public University Corporation Nagoya City University, Mizuho-ku Nagoya-shi Aichi 467-8601 (JP); Hayashibara Co., Ltd., Okayama-shi, Okayama 702-8006 (JP)
(72) Inventor: INAGAKI, Hiroshi, Nagoya-shi, Aichi 467-8601 (JP); TAKASE, Hiroshi, Nagoya-shi, Aichi 467-8601 (JP); KUSANO, Hajime, Okayama-shi, Okayama 702-8006 (JP); NAGAIKE, Hiroshi, Okayama-shi, Okayama 702-8006 (JP); ARIYASU, Toshio, Okayama-shi, Okayama 702-8006 (JP)
(74) Representative: Nederlandsch Octrooibureau
(86) International application number: PCT/JP2021/014173
(87) International publication number: WO 2021/201233

(57) **Abstract**

A staining method includes staining a biological sample with a coumarin fluorescent dye to provide a fluorescent-stained sample, and bringing the fluorescent-stained sample into contact with osmium tetroxide, further embedding the sample in an epoxy resin, and subsequently slicing the sample to provide a section sample including the fluorescent-stained sample.

## Description

### Technical Field

The present invention relates to a staining method, a microscopic observation method, a staining agent, and a staining kit. The present application claims priority based on Japanese Patent Application No. 2020-066711 filed on April 2, 2020, and the contents of the Japanese patent application are incorporated herein by reference.

### Background Art

Correlative light and electron microscopy method (CLEM method) in which observation with a fluorescence microscope and observation with an electron microscope are used in combination has been used to date as a method for observing living tissue or a microstructure such as a cell organelle or a membrane structure of a microorganism or the like (for example, PTL 1).

In general, fluorescent dyes used for staining have low resistance to preliminary treatment for electron microscopic observation, using, for example, osmium tetroxide and an epoxy compound. Therefore, when a section sample stained with a fluorescent dye is subjected to osmium treatment and epoxy-resin embedding in order to observe the section sample with an electron microscope, there is a problem in that the fluorescent dye in the section sample is subjected to a structural change, resulting in disappearance of fluorescence.

For this reason, when the CLEM method is performed, both fluorescence-staining treatment for fluorescence observation and preliminary treatment for electron microscopic observation cannot be performed for the identical section sample. Thus, after fluorescence microscopic observation of a section sample that has been subjected to fluorescence staining is performed, it is necessary to perform osmium treatment and epoxy resin embedding treatment for the section sample in order to further observe the section sample with an electron microscope. However, such an existing method has a problem in that the section sample for electron microscopic observation, the section sample having been subjected to the osmium treatment and the epoxy resin embedding treatment, is not assured to be in the same state as the section sample having been subjected to fluorescence observation.

### Citation List

### Patent Literature

PTL 1: Japanese Unexamined Patent Application Publication No. 2017-006070

### Summary of Invention

### Technical Problem

The present invention provides a staining method in which a single section sample is subjected to fluorescence staining, osmium treatment, and epoxy resin embedding treatment in advance and can then be subjected to fluorescence microscopic observation and electron microscopic observation, a method for microscopic observation of a section sample obtained by the staining method, and a staining agent and a staining kit that are useful for the staining method.

### Solution to Problem

The inventors of the present invention have found that a coumarin fluorescent dye is unlikely to be decomposed even when subjected to osmium tetroxide treatment and epoxy resin embedding treatment and completed the present invention.
[1] A staining method comprising staining a biological sample with a coumarin fluorescent dye represented by formula (I) below to provide a fluorescent-stained sample; and bringing the fluorescent-stained sample into contact with osmium tetroxide, further embedding the sample in an epoxy resin, and subsequently slicing the sample to provide a section sample including the fluorescent-stained sample.
[2] The staining method according to [1], comprising oxidation treatment, wherein the biological sample is subjected to oxidation treatment in advance to change at least some of hydroxyl groups of a polysaccharide contained in the biological sample to aldehyde groups.
[3] The staining method according to [2], comprising binding the coumarin fluorescent dye to some of the aldehyde groups formed in the polysaccharide to provide the fluorescent-stained sample.
[4] The staining method according to [1], comprising binding a primary antibody having the coumarin fluorescent dye to the biological sample.
[5] The staining method according to [1], comprising binding a primary antibody to the biological sample, and subsequently indirectly binding the coumarin fluorescent dye to the primary antibody.
[6] The staining method according to [5], wherein the primary antibody has biotin, and avidin or streptavidin having a plurality of the coumarin fluorescent dyes is bound to the biotin.
[7] The staining method according to [1], comprising binding a primary antibody or secondary antibody having a peroxidase to the biological sample, converting a tyramide compound having the coumarin fluorescent dye into a radical by a catalytic action of the peroxidase in the presence of hydrogen peroxide, and binding the tyramide compound to a protein present in the vicinity of the primary antibody or the secondary antibody to provide the fluorescent-stained sample.
[8] A microscopic observation method comprising observing a section sample obtained by the staining method according to any one of [1] to [7] with both a fluorescence microscope and an electron microscope.
[9] The microscopic observation method according to [8], comprising superimposing an image obtained by observation with the fluorescence microscope and an image obtained by observation with the electron microscope to obtain an image.
[10] A staining agent comprising a coumarin fluorescent dye represented by formula (I) below, the staining agent being used in an application for staining a biological sample provided for electron microscopic observation.
[11] The staining agent according to [10], wherein the biological sample is also provided for fluorescence microscopic observation after being stained.
[12] A staining agent comprising a labeled substance to which a reactive group of a coumarin fluorescent dye represented by formula (I) below is chemically bound, the staining agent being used in an application for staining a biological sample provided for electron microscopic observation.
[13] The staining agent according to [12], wherein the labeled substance is at least one selected from the group consisting of an antibody, avidin, streptavidin, and a tyramide compound.
[14] The staining agent according to [12] or [13], wherein the biological sample is also provided for fluorescence microscopic observation after being stained.
[15] A staining kit comprising the staining agent according to any one of [10] to [14], the staining kit being used in an application for staining a biological sample provided for electron microscopic observation.
[16] The staining kit according to [15], comprising the staining agent containing at least one labeled substance according to any one of [12] to [14]; and at least one reagent that indirectly binds the labeled substance to any primary antibody that binds to the biological sample.
[17] The staining kit according to [16], comprising, as the reagent, a secondary antibody that has biotin and that binds to the primary antibody; and, as the labeled substance, avidin or streptavidin to which the reactive group of the coumarin fluorescent dye is chemically bound.
[18] The staining kit according to [16], comprising, as the reagent, a secondary antibody that has a peroxidase and that binds to the primary antibody; and, as the labeled substance, a tyramide compound to which the reactive group of the coumarin fluorescent dye is chemically bound, and hydrogen peroxide.
[19] The staining kit according to any one of [15] to [18], wherein the biological sample is also provided for fluorescence microscopic observation after being stained. Advantageous Effects of Invention

According to the staining method of the present invention, for a biological sample provided for the CLEM method, subsequent to fluorescence staining using a coumarin fluorescent dye, osmium treatment and epoxy resin embedding treatment can be further performed. Thus, fluorescence microscopic observation and electron microscopic observation can be continuously performed for the identical section sample without performing treatment necessary for the existing CLEM method (such as osmium treatment and epoxy resin embedding treatment performed for a sample that has already been used for fluorescence microscopic observation).

### Brief Description of Drawings

[Fig. 1] Fig. 1 is an image A1 obtained by observing, with a fluorescence microscope, a stained section sample obtained in Example 1.
[Fig. 2] Fig. 2 is an image B1 obtained by observing, with an electron microscope, the stained section sample obtained in Example 1.
[Fig. 3] Fig. 3 is an image C1 obtained by superimposing the image A1 and the image B1 obtained in Example 1.
[Fig. 4] Fig. 4 is an image A2 obtained by observing, with a fluorescence microscope, a stained section sample obtained in Example 2.
[Fig. 5] Fig. 5 is an image B2 obtained by observing, with an electron microscope, the stained section sample obtained in Example 2.
[Fig. 6] Fig. 6 is an image C2 obtained by superimposing the image A2 and the image B2 obtained in Example 2.
[Fig. 7] Fig. 7 is a graph showing the results of Reference Experiment 1.
[Fig. 8] Fig. 8 is a graph showing the results of Reference Experiment 2.
[Fig. 9] Fig. 9 is a graph showing the results of Reference Experiment 3.
[Fig. 10] Fig. 10 is a graph showing the results of Reference Experiment 4.
[Fig. 11] Fig. 11 is a graph showing the results of Reference Experiment 5.
[Fig. 12] Fig. 12 is a graph showing the results of Reference Experiment 6.
[Fig. 13] Fig. 13 is a graph showing the results of Reference Experiment 7.
[Fig. 14] Fig. 14 is a graph showing the results of Reference Experiment 8.
[Fig. 15] Fig. 15 is a graph showing the results of Reference Experiment 9.
[Fig. 16] Fig. 16 is a graph showing the results of Reference Experiment 10.
[Fig. 17] Fig. 17 is a graph showing the results of Reference Experiment 11.
[Fig. 18] Fig. 18 is a graph showing the results of Reference Experiment 12.
[Fig. 19] Fig. 19 is a graph showing the results of Reference Experiment 13.
[Fig. 20] Fig. 20 is a graph showing the results of Reference Experiment 14.
[Fig. 21] Fig. 21 is an observation image A3 of a section sample subjected to immunofluorescence staining in Example 3.
[Fig. 22] Fig. 22 is an observation image B3 of a section sample subjected to DAB staining in Comparative Example 1.
[Fig. 23] Fig. 23 is an image C3 obtained by superimposing the observation image A3 obtained in Example 3 and the observation image B3 obtained in Comparative Example 1.

### Description of Embodiments

### <<Staining Object>>

A staining method according to the present invention is applicable to biological samples such as cells, living tissue, microorganisms, and viruses. In order to observe a stained sample by the CLEM method, the sample needs to be prepared as a section with a thickness suitable for electron microscopic observation. The sample provided for the staining method according to the present invention may be prepared, in advance, as a section such as a paraffin-embedded section or a frozen section or may be prepared as a section by a routine method after being subjected to fluorescence staining described later.

When a paraffin-embedded section is used as a staining object, before fluorescence staining, deparaffinization treatment is preferably performed by, for example, a publicly known method in which the paraffin-embedded section is sequentially immersed in xylene, ethanol, and ion-exchanged water.

### <<Staining Method>>

A first embodiment of the present invention is a staining method including a fluorescent staining step and an epoxy resin embedding step.

The fluorescent staining step is a step of staining a biological sample with a coumarin fluorescent dye represented by formula (I) below to provide a fluorescent-stained sample.

The epoxy resin embedding step is a step of bringing the fluorescent-stained sample into contact with osmium tetroxide, further embedding the sample in an epoxy resin, and subsequently slicing the sample to provide a section sample including the fluorescent-stained sample. Each of the steps will be described below.

### <Fluorescent Staining Step>

In this step, a stain solution containing a coumarin fluorescent dye and a biological sample, which is a staining object, are brought into contact with each other to bind the coumarin fluorescent dye to a target substance contained in the biological sample either directly or indirectly.

A coumarin fluorescent dye represented by formula (I) below may be hereinafter referred to as a compound (I).

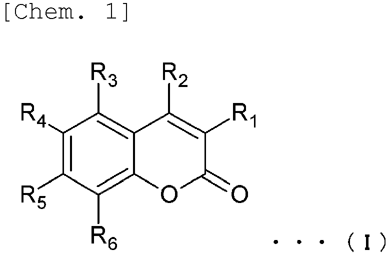

In formula (I), R₁ to R₆ are each independently a hydrogen atom or any substituent,
at least one selected from R₁, R₂, and R₅ includes a reactive group,
the reactive group is a functional group capable of forming a chemical bond with an aldehyde, an amine, or a thiol,
R₁ may have an aromatic ring, and one or more hydrogen atoms bound to the aromatic ring may be substituted with the reactive group through a linker,
when the aromatic ring is an aromatic heterocycle having a heteroatom other than a carbon atom, the reactive group may be bound to the heteroatom through a linker, and
R₄ and R₆ may each independently form a ring together with R₅.

The aromatic ring may be a monovalent or divalent monocyclic ring or a monovalent or divalent polycyclic ring. The aromatic ring may be a monovalent or divalent aromatic hydrocarbon or a monovalent or divalent aromatic heterocycle.

Of the reactive groups, the functional group capable of forming a chemical bond with an aldehyde is, for example, an amino group (-NH₂), a hydrazide group (-CONH-NH₂), or an aminoxy group (-O-NH₂).

Of the reactive groups, the functional group capable of forming a chemical bond with an amine is, for example, an N-hydroxysuccinimidyl group (NHS group) represented by formula (Q-1) below and an imidoester group represented by formula (Q-2) below.

Of the reactive groups, the functional group capable of forming a chemical bond with a thiol is, for example, a maleimide group represented by formula (Q-3) below. 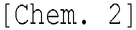 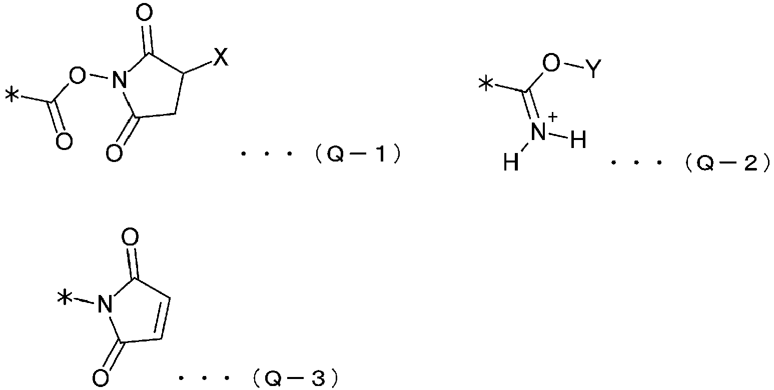 [In the formulae, * represents binding to another atom, X represents a hydrogen atom or a sulfonate (e.g., Na sulfonate), and Y represents a methyl group or an ethyl group.]

The reactive group may be directly bound to the coumarin ring structure to which R₁ to R₆ are bound or may be indirectly bound to the coumarin ring structure through a linker.

The linker is a divalent organic group. The linker can link the reactive group to the coumarin ring structure either directly or indirectly.

Examples of the linker include linear aliphatic hydrocarbon groups having 1 to 10 carbon atoms.

One or more methylene groups constituting any of the aliphatic hydrocarbon groups, except when oxygen atoms are bound to each other, may be substituted with a divalent substituent such as an ether group (-O-), a thioether group (-S-), an ester group (-CO-O-), a carbonyl group (-C(=O)-), a carboxamide group (-CO-NH-), a sulfonyl group (-SO₂-), a sulfonamide group (-SO₂-NH-) or a hydrazide group (-CONH-NH-) .

One or more methylene groups constituting any of the aliphatic hydrocarbon groups may be substituted with a cyclic aliphatic hydrocarbon group having 5 to 8 carbon atoms. This cyclic group is a divalent group formed by removing any two hydrogen atoms bound to a cyclic aliphatic hydrocarbon. A trivalent carbon atom (-CH<) which constitutes this cyclic group and from which the hydrogen atom has been removed may be substituted with a nitrilo group (-N<).

In formula (I), when one or more of R₁ to R₆ are each any substituent, the substituents may each be independently, for example, a linear or branched aliphatic saturated hydrocarbon group having 1 to 10 carbon atoms, a linear or branched aliphatic unsaturated hydrocarbon group having 1 to 8 carbon atoms, a sulfonic group (-SO₃H), a carboxylic group (-COOH), an acetyl group (-COCH₃), an alkoxy group having 1 to 4 carbon atoms, a halogen atom, or a hydroxyl group. Furthermore, as any substituent may be a substituent in which one or more methylene groups constituting the aliphatic saturated hydrocarbon group or the aliphatic unsaturated hydrocarbon group, except when oxygen atoms are bounded to each other, are each substituted with -O-, - C(=O)-, -C(=O)O-, -O-C(=O)-, -SO₂- or -NH-.

When the substituent is an acid group having a negative charge, the acid group may form a salt such as a sodium salt or a potassium salt. When the substituent has a positive charge (for example, has a nitrogen atom constituting a quaternary ammonium), the positive charge may form a salt with any counter anion or may form an inner salt with any other substituent having a negative charge. A hydrogen atom of the substituent may be substituted with a halogen atom.

Preferably, the substituent does not include a carboxylic group having a negative charge. When the acid group is not included, it is possible to prevent the acid group from reacting with an epoxy compound provided from the outside.

In formula (I), one or more hydrogen atoms bound to the aromatic ring included in R₁ may be substituted with substituent R₁₁. Substituent R₁₁ has a linker which is a divalent organic group and the described reactive group bound to an end of this linker. Examples of the linker of substituent R₁₁ include the divalent organic groups mentioned. One or more hydrogen atoms bound to the aromatic ring may be substituted with any substituent other than substituent R₁₁.

Substituent R₁₁ may be, for example, -(CH₂)ᵣ-Q. Here, Q represents any of the above reactive groups and r represents an integer of 0 to 10. Each methylene group constituting substituent R₁₁ may be substituted with -O-, -C(=O)-, - C(=O)O-, -O-C(=O)-, -NH-, -SO₂-, or -S- excepted oxygen atoms are bound to each other.

In formula (I), when a plurality of substituents R₁₁ are present, the substituents R₁₁ may be the same or different from each other.

The compound (I) is preferably a compound represented by the following formulae (I-A) to (I-H) from the viewpoint that it sufficiently exhibits resistance to osmium treatment and epoxy resin embedding treatment and the fluorescence characteristics are excellent. In formulae below, Q represents the reactive group, L represents the linker, and Ar represents a divalent aromatic ring.

An "-L-Q" group in formula (I-G) below may replace any hydrogen atom of Ar or may be bound to a heteroatom constituting Ar. In the latter case, the heteroatom may have a positive charge, and (Z⁻) represents any counter anion to this positive charge. In the former case, (Z⁻) need not be present. Alternatively, the heteroatom having a positive charge and any acid group in the molecule may form an inner salt. Also in such a case, (Z⁻) need not be present.

In formula (I-H) below, Ar represents an aromatic heterocycle having a nitrogen atom, and an "-L-Q" group is bound to this nitrogen atom. The nitrogen atom has a positive charge, and (Z⁻) represents any counter anion.

Examples of the counter anion include monovalent anions such as a halide ion, a sulfonate anion, a hexafluorophosphate anion, a tetrafluoroborate anion, a p-chlorobenzenesulfonate anion, a p-toluenesulfonate anion, a benzenesulfonate anion, a trifluoromethanesulfonate anion, and a trifluoroacetate anion. The nitrogen atom having a positive charge and any acid group in the molecule may form an inner salt. In such a case, (Z⁻) need not be present.

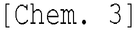

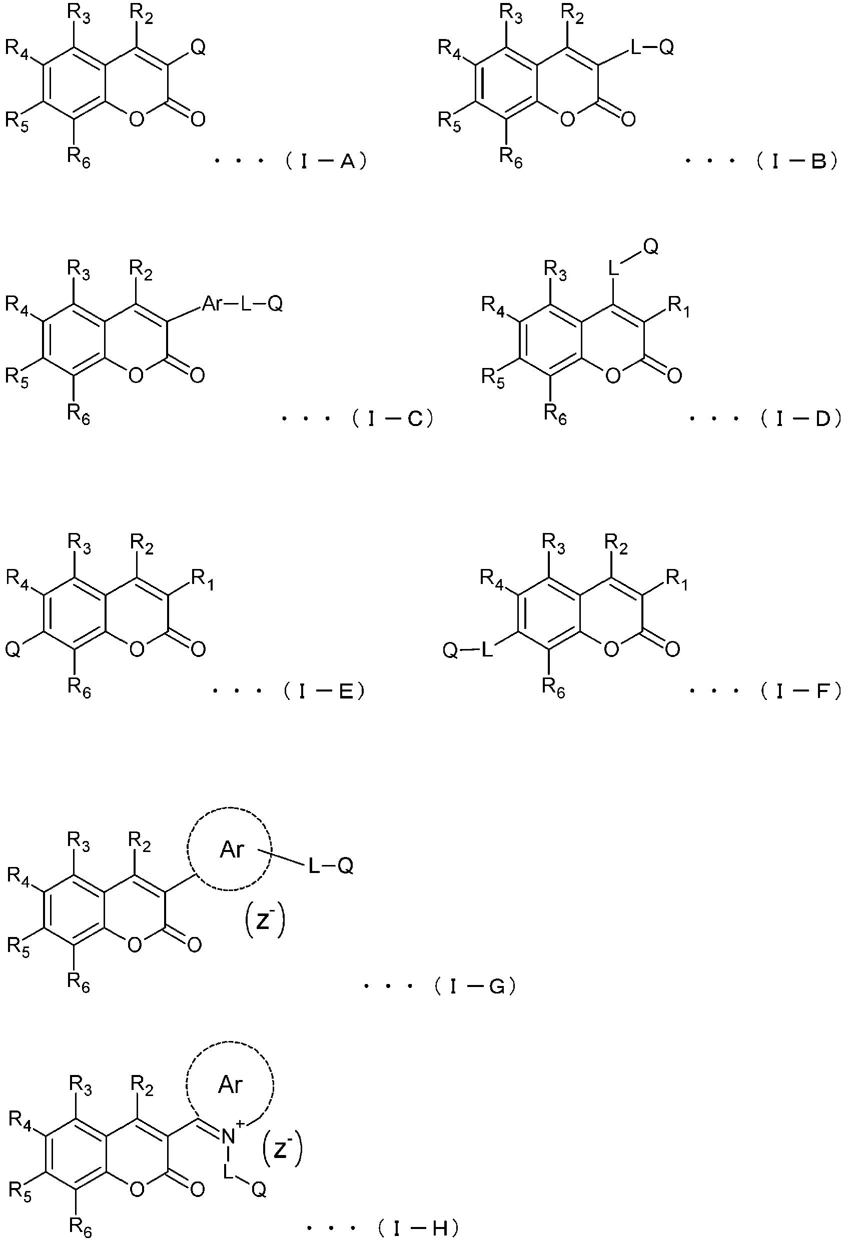

Suitable specific examples of the compound (I) include compounds represented by formulae (1) to (17) below. Compounds represented by Formulae (1) and (4) below are NKX-4023 and NKX-4190, respectively, used in Examples described later.

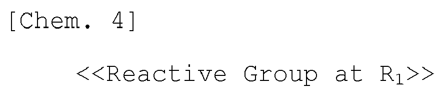

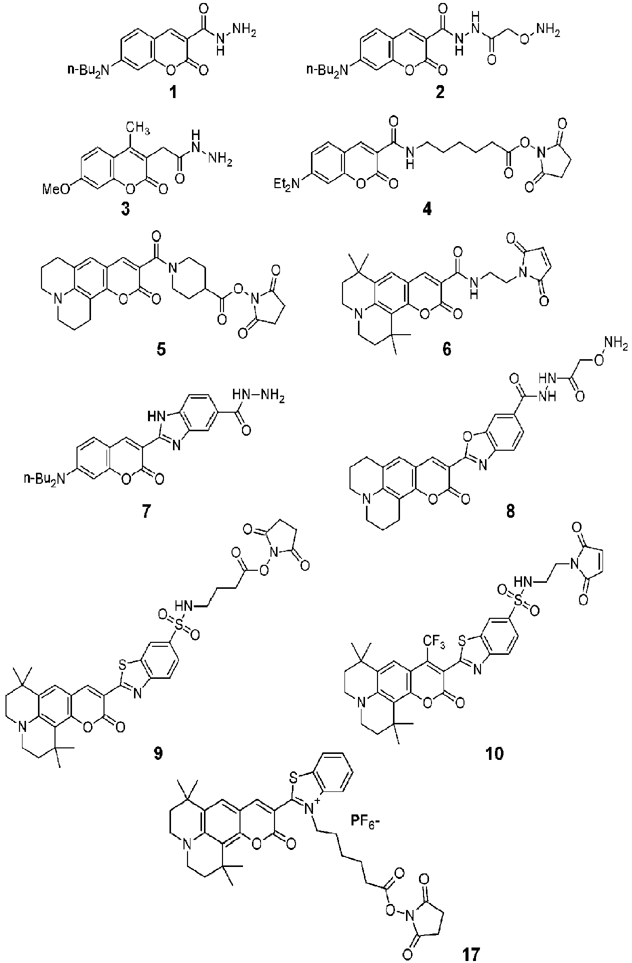

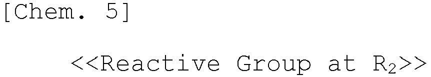

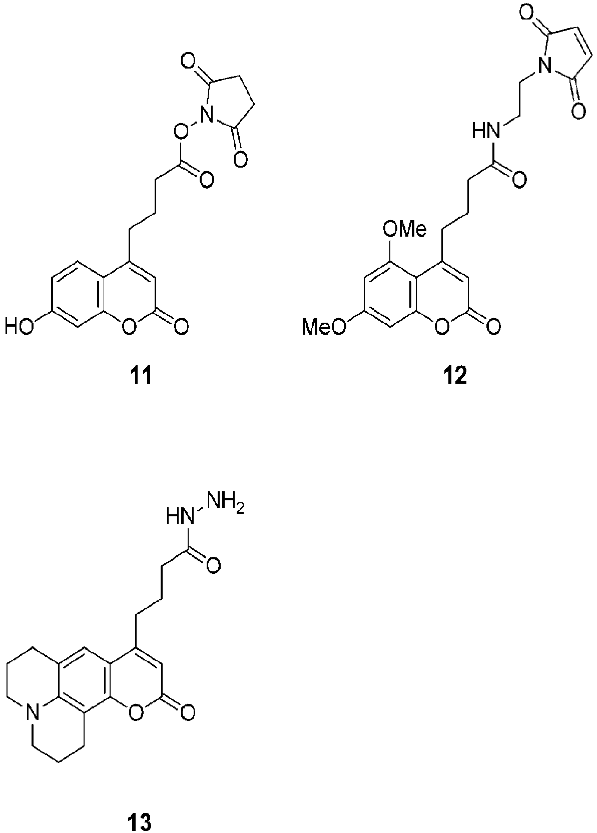

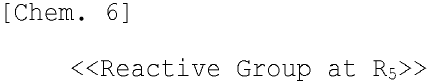

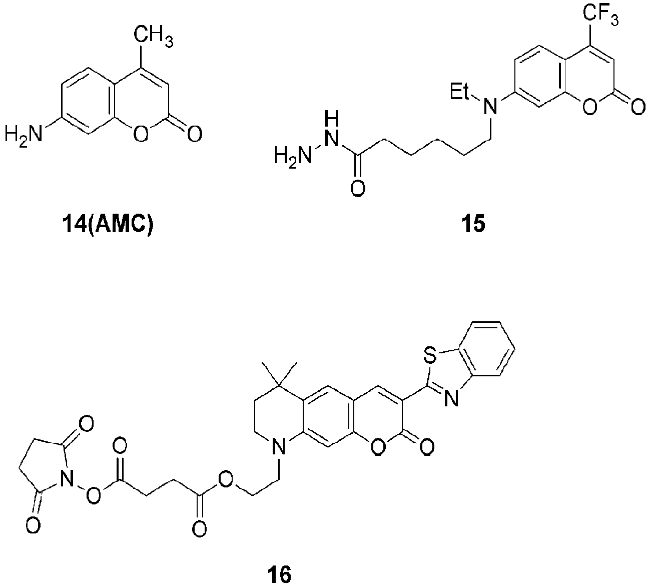

Reference compounds represented by formulae (101) to (112) below do not correspond to the compound (I) in that they do not have the reactive group; however, the reference compounds have a coumarin ring structure similar to that of the compound (I). Accordingly, by binding the reactive group to any of the reference compounds shown as examples here or another reference compound having a similar coumarin ring structure, the compound (I) according to the present invention can be provided. The reactive group may be directly bound to the reference compound or may be bound via the linker. The reactive group and the linker that is optionally interposed may be bound to the reference compound by replacing a hydrogen atom of the reference compound or may be bound to a heteroatom of the reference compound.

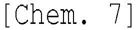

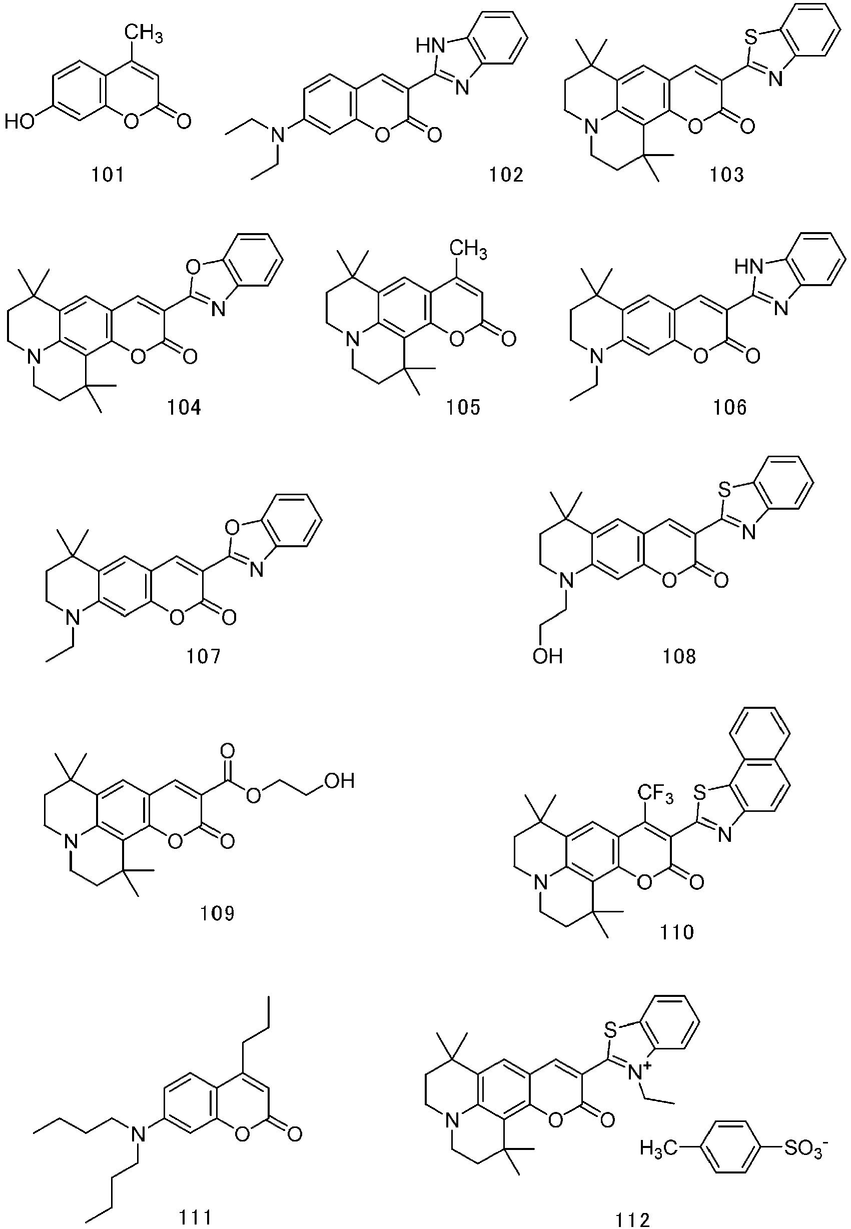

An example of a method of binding the reactive group to a heteroatom of the reference compound through the linker is a synthesis method represented by reaction formula below.

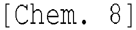

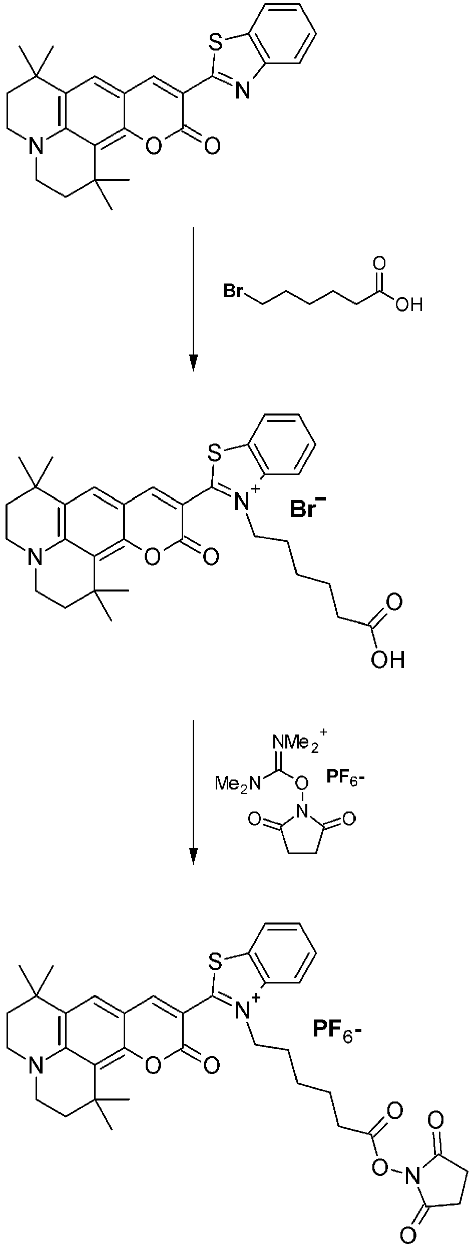

When a stain solution containing at least one compound (I) is brought into contact with a biological sample, which is a staining object, to perform staining (hereinafter referred to as fluorescence staining), the compound (I) can be bound to a desired substance contained in the biological sample depending on the type of the reactive group.

When a reactive group of the compound (I) binds to an aldehyde group, the compound (I) can be bound to a substance having an aldehyde group and contained in a biological sample.

Even for a substance that does not originally have an aldehyde group, a biological sample may be subjected to preliminary treatment in advance to form an aldehyde group in a desired molecule in advance. For example, when cells (adenocytes) that produce mucus containing a polysaccharide are subjected to fluorescence staining, a preliminary treatment may be performed by treating a tissue section including the cells with periodic acid to oxidize the polysaccharide contained in the cells, thereby oxidizing hydroxyl groups in the molecule of the polysaccharide to aldehyde groups. This preliminary treatment is publicly known as treatment in PAS staining.

The polysaccharide is not particularly limited as long as the polysaccharide is oxidized by an oxidizing agent to produce an aldehyde group, and examples thereof include polysaccharides having glucose as a constitutional unit. Specific examples thereof include glycogen, mucus proteins, glycoproteins, and glycolipids. The glycoproteins also include antibodies in which the Fc region is modified with a polysaccharide.

After the substance having an aldehyde group and contained in the biological sample and the compound (I) are bound together by fluorescence staining with Schiff reaction, a reductive amination reagent may be used in order to stabilize the formed bond (Schiff base). An example of the reductive amination reagent is 2-picoline borane. The reductive amination reagent can be added in an appropriate concentration in advance to the compound (I)-containing stain solution used in fluorescence staining.

When a reactive group of the compound (I) binds to an amino group, the compound (I) can be bound to a substance having an amino group in a biological sample. Typical examples of the substance having an amino group include proteins having a lysine (Lys) residue or an arginine (Arg) residue.

When a biological sample is contacted with a stain solution containing the compound (I) having a reactive group that binds to an amino group, the compound (I) is non-specifically bound to a protein contained in the biological sample. When binding the compound (I) to only the target protein, it is preferable to use an antibody that binds to the target protein. That is, a labeled antibody is prepared by binding in advance the compound (I) to an amino group of an antibody to be used as a reagent, and the labeled antibody is bound to the target protein contained in a biological sample by using the antigenic specificity of this labeled antibody. Thus, the compound (I) can be bound to the target protein through the labeled antibody.

When a reactive group of the compound (I) binds to a thiol group, the compound (I) can be bound to a substance having a thiol group in a biological sample. Typical examples of the substance having a thiol group include proteins having a cysteine (Cys) residue or a disulfide bond (-S-S-). The disulfide bond can be converted to a thiol group by reducing with dithiothreitol (DTT) or β-mercaptoethanol.

When a stain solution containing the compound (I) having a reactive group that binds to a thiol group is brought into contact with a biological sample, the compound (I) is non-specifically bound to a protein contained in the biological sample. Thereby, when it is desired to bind the compound (I) to only the target protein, an antibody that binds to the target protein is preferably used. That is, a labeled antibody is prepared by binding in advance the compound (I) to a thiol group of an antibody to be used as a reagent, and the labeled antibody is bound to the target protein contained in a biological sample by using the antigenic specificity of this labeled antibody. Thus, the compound (I) can be bound to the target protein through the labeled antibody.

As an example of fluorescence staining, for example, a primary labeled antibody in which a first compound (I) is bound to a primary antibody and a secondary labeled antibody in which a second compound (I) is bound to a secondary antibody are prepared respectively, and these two kinds of labeled antibodies can be used for multiple fluorescent staining of the sample. In this case, the fluorescence wavelengths of each compound (I) are preferably different, and the antigen specificities of each antibody is preferably also different.

In the fluorescence staining for labeling an antibody with the compound (I), the antibody that can be labeled with the compound (I) may be a primary antibody, or a secondary antibody, and furthermore, it may be other molecules that specifically binds to a primary antibody either directly or indirectly.

### [Chemiluminescence Amplification Method]

The compound (I) is also applicable to a labeling system that uses specific binding properties of avidin and biotin (avidin-biotin system). For example, a secondary antibody is labeled with biotin in advance, and avidin to be bound to this secondary antibody is labeled with the compound (I) in advance. Thus, a complex of "antigen + primary antibody + secondary antibody + plural biotin molecules + plural avidin molecules + plural molecules of the compound (I)" is formed, and the target antigen can be subjected to fluorescence staining. By labeling each secondary antibody molecule with a plurality of biotin molecules, a plurality of avidin molecules can be bound to each secondary antibody molecule. In this case, by labeling each avidin molecule with a plurality of molecules of the compound (I), as a result, a large number of molecules of the compound (I) can be indirectly bound to the target antigen.

In the above method, streptavidin may be used instead of avidin.

The compound (I) is also applicable to a signal amplification system using an enzyme, which is called a catalyzed reporter deposition (CARD) method. For example, a CARD method using a peroxidase derived from horseradish (HRP) and a tyramide substrate is known. Specifically, when a secondary antibody is labeled with HRP, and a tyramide substrate is added in the presence of hydrogen peroxide, which is a substrate of HRP, the tyramide substrate is converted into a highly reactive radical intermediate to non-specifically form a covalent bond with a tyrosine residue or a tryptophan residue in proteins present in the vicinity of the secondary antibody. That is, by accumulating a large number of molecules of the tyramide substrate labeled with the compound (I) in the vicinity of a complex of "antigen + primary antibody + secondary antibody + HRP", fluorescence staining can be performed on or near the target antigen.

As a simple method of fluorescence staining, for example, a section of a biological sample is immersed in a stain solution containing at least one compound (I) in any concentration at room temperature for about 15 to 60 minutes and then taken out, and excessive compounds are washed with water.

The total concentration of the compound (I) contained in the stain solution may be the same as a concentration of a fluorescent dye used in existing fluorescence staining and is, for example, 0.01 g/L to 0.2 g/L.

When the compound (I) is bound in advance to an antibody, avidin, a tyramide substrate, or the like, the concentration of the compound (I) contained in the stain solution is appropriately determined according to the concentration of the antibody, avidin, tyramide substrate, or the like.

From the viewpoint of increasing the efficiency of fluorescence staining, the biological sample is preferably an individually separated sample prepared by separating a sample into individual pieces (blocks) in advance or a sliced section sample. The thickness of the section sample can be, for example, about 1 µm to 50 µm. A sectioning of the biological sample is carried out by a routine method, for example, the method in which the biological sample is embedded in paraffin or frozen and then sliced with a microtome or the like.

The fluorescent-stained sample prepared by subjecting a biological sample to fluorescence staining in this step is provided to the following epoxy resin embedding step.

### <Epoxy Resin Embedding Step>

In this step, the fluorescent-stained sample obtained in the previous step is subjected to publicly known fixation treatment and resin embedding treatment that are performed for a sample to be observed with a transmission electron microscope.

First, the fluorescent-stained sample may be brought into contact with a reducing agent solution containing glutaraldehyde or formaldehyde to perform prefixation. This prefixation is an optional treatment, and can be fixed by denaturing the protein contained in the fluorescent-stained sample. In the case where, for example, an antibody or avidin is used in fluorescence staining in the previous step, the antibody or avidin bound to an antigen or biotin can be fixed. In this case, the compound (I) having a coumarin ring skeleton can maintain its chemical structure to such an extent that fluorescence observation can be performed later.

Note that prefixation is an optional treatment, and osmium tetroxide treatment may be performed without prefixation, but the cellular structures can be sufficiently maintained by performing prefixation, and the accuracy of electron microscopic observation can be improved.

The prefixation may be performed at about 4°C for about one hour to one night.

Next, the fluorescent-stained sample is brought into contact with an oxidizing agent solution containing osmium tetroxide to perform postfixation. When postfixation is performed subsequent to the prefixation, cellular structures can be sufficiently maintained to improve the accuracy of electron microscopic observation.

The postfixation may be performed at a temperature of cooling with ice (0°C to 4°C) for about 15 minutes to one hour.

Subsequent to the postfixation, heavy metal staining using uranium is preferably performed. Specifically, the fluorescent-stained sample that has been subjected to postfixation is preferably brought into contact with a heavy metal solution containing uranyl acetate to perform staining. Since a heavy metal preferentially adsorbs to proteins and some of lipids contained in the fluorescent-stained sample, the contrast of these can be enhanced in electron microscopic observation.

The fluorescent-stained sample may be subjected to lead staining instead of uranyl acetate staining or in addition to uranyl acetate staining. Specifically, the lead staining can be performed by a routine method.

Subsequently, the fluorescent-stained sample that has been subjected to heavy metal staining is brought into contact with a water-soluble organic solvent such as ethanol or acetone to dehydrate water contained in the fluorescent-stained sample. In a method, for example, the fluorescent-stained sample is first brought into contact with a 50% to 70% aqueous ethanol solution, and the aqueous ethanol solution is gradually substituted with aqueous ethanol solutions having increased ethanol concentrations and finally substituted with 100% ethanol. Furthermore, a substitution with a liquid epoxy compound such as oxidized propylene (propylene oxide) or an epoxy resin is performed to substitute water contained in the fluorescent-stained sample with the epoxy compound or the epoxy resin, and the epoxy compound or the epoxy resin is then cured by thermal polymerization at about 60°C. As a result, an embedded sample in which the fluorescent-stained sample is fixed and embedded in an epoxy resin is obtained.

The embedded sample is a piece of epoxy resin and thus can be sliced into, by a routine method, a section having a thickness suitable for transmission electron microscopic observation, for example, 50 nm to 120 nm.

The section sample obtained in the steps described above has been subjected to both fluorescence staining for fluorescence observation and osmium treatment for electron microscopic observation.

The compound (I) bound to the section sample is excited by light and then emits fluorescence. Observation of this fluorescence with a fluorescence microscope or the like enables examination of, for example, the distribution of a substance having an aldehyde group in the section sample and the localization of an antigen that specifically binds to an antibody.

The wavelength of fluorescence emitted by the compound (I) is dominated by the coumarin ring structure of the compound (I), and the fluorescence wavelength changes depending on the substituent. For example, the compound (I) is excited at a wavelength of around 320 nm to 530 nm, at a wavelength of around 340 nm to 450 nm, or at a wavelength of around 365 nm to 405 nm and can be observed as fluorescence with a wavelength in the visible region.

Since a heavy metal such as osmium or uranium that binds to the section sample changes the direction of an electron beam applied to the section sample, regions of the section sample where the heavy metal adsorbs are detected as shadow-like dark regions through which a less number of electrons transmit. On the other hand, since light atoms such as hydrogen, carbon, oxygen, and nitrogen that constitute a living body do not significantly affect the transmission of electrons, regions where these atoms are present are detected as bright regions through which a large number of electrons transmit. Consequently, this provides an image in which proteins and some of lipids to which the heavy metal is likely to adsorb are observed as a shadow.

In the staining method according to the present invention, since the compound (I) is used as a fluorescent labeling substance, fluorescence observation can be sufficiently performed even after the treatment for electron microscopic observation is performed. Therefore, observation with a fluorescence microscope and observation with an electron microscope can be continuously performed for the identical section sample. For example, the publicly known CLEM method can be applied.

The feature that both fluorescence microscopic observation and electron microscopic observation can be continuously performed for the identical section sample is an extremely significant advantageous in the field of pathological examinations. That is, in a section sample to be subjected to a pathological examination, for the site focused by observation with a fluorescence microscope, the same site of the same section can be observed with an electron microscope without changing the section sample.

In existing pathological examinations, a section sample is first subjected to fluorescence staining, and if a position to be carefully examined is specified, the section sample is subjected to fixation treatment and epoxy resin embedding treatment, and a thinner section is then cut out in order to perform electron microscopic observation. In this existing method, since the thickness of the section sample that has been subjected to fluorescence observation and the thickness of the section sample that has been subjected to electron microscopic observation are different from each other, and shear stress and torsional stress are applied when the section is thinly cut out, a physical state other than the thickness also changes in a precise sense. Therefore, there may occur a situation where a substance or biological structural information included in the section sample for fluorescence observation is not included in the section sample for an electron microscope. Accordingly, there is a concern that accurate pathological diagnosis is not necessarily performed by the existing method.

### <<Microscopic Observation Method>>

A second embodiment of the present invention is a microscopic observation method including observing a section sample obtained by the staining method of the first embodiment with both a fluorescence microscope and an electron microscope.

A publicly known fluorescence microscope including a light source that excites the compound (I) and a detector that receives fluorescence of the compound (I) is applicable to the fluorescence microscope used. A publicly known transmission electron microscope is applicable to the electron microscope used. A publicly known method is applicable to a specific observation method.

Regarding the order of observation of the section, after fluorescence microscopic observation is performed, electron microscopic observation may be performed, after electron microscopic observation is performed, fluorescence microscopic observation may be performed, or fluorescence microscopic observation and electron microscopic observation may be simultaneously performed if a device that can simultaneously perform fluorescence microscopic observation and electron microscopic observation is provided. For example, in an observation method, fluorescence microscopic observation is performed at a relatively low magnification to specify a position to be observed in more detail, and the specified position is then subjected to electron microscopic observation.

When an image obtained by fluorescence microscopic observation and an image obtained by electron microscopic observation are superimposed, the superimposed image provides information related to the distribution and localization of the compound (I) in the electron microscopic image. The superimposition of the images is performed by making observation regions (coordinates) of the images coincide. Publicly known image processing is applicable to specific superimposition of the images.

In the microscopic observation method described above, the operation of the microscopes and the image processing can be carried out as in the publicly known CLEM method.

In the existing CLEM method, both fluorescence microscopic observation and electron microscopic observation cannot be performed for an identical section sample. In contrast, according to the present invention, both fluorescence microscopic observation and electron microscopic observation can be performed for an identical section sample without adding any additional treatment.

### <<Staining Agent>>

A third embodiment of the present invention is a staining agent containing a coumarin fluorescent dye (compound (I)) represented by the formula (I). The staining agent of this embodiment is used in an application for staining a biological sample provided for electron microscopic observation.

A fourth embodiment of the present invention is a staining agent containing a labeled substance to which a reactive group of a coumarin fluorescent dye (compound (I)) represented by the formula (I) is chemically bound. The staining agent of this embodiment is used in an application for staining a biological sample provided for electron microscopic observation.

By using any of the staining agents of the third and fourth embodiments, the staining method of the first embodiment and the microscopic observation method of the second embodiment can be performed.

The description of the compound (I) of the third and fourth embodiments is the same as the description of the compound (I) of the first embodiment, and redundant description is thus omitted here.

The compound (I) contained in the staining agent may be one compound or two or more compounds.

The form of the staining agent may be a solid such as a powder or tablet containing the compound (I) or a liquid in which the compound (I) is dissolved or dispersed in any solvent at any concentration.

The solvent is not particularly limited as long as the solvent can dissolve or disperse the compound (I). Examples of the solvent include purified water, primary alcohols such as methanol, ethanol, and isopropanol, and other organic solvents such as acetonitrile, DMSO, and hexane.

In the fourth embodiment, examples of the labeled substance to which a reactive group of the compound (I) is chemically bound include a substance in which at least one compound (I) is chemically bound to a primary antibody or a secondary antibody, a substance in which at least one compound (I) is chemically bound to avidin or streptavidin, and a substance in which at least one compound (I) is chemically bound to a tyramide substrate, as described above.

### <<Staining Kit>>

A fifth embodiment of the present invention is a staining kit including the staining agent of the third embodiment or the fourth embodiment.

Specifically, examples thereof include the following staining kit A and staining kit B.

The staining kit A is a staining kit for a biological sample provided for electron microscopic observation, the staining kit including the staining agent of the third embodiment, and at least one reagent that directly or indirectly binds the compound (I) to any primary antibody that binds to a biological sample.

The staining kit B is a staining kit including the staining agent of the fourth embodiment, and at least one reagent that indirectly binds the labeled substance contained in the staining agent to any primary antibody that binds to a biological sample.

The staining kit B preferably includes, as the reagent, a secondary antibody that has biotin and that binds to the primary antibody, and avidin or streptavidin to which the reactive group of the compound (I) is chemically bound. The phrase "secondary antibody has biotin" means that the secondary antibody is labeled with biotin, that is, biotin is bound to the secondary antibody. The binding between biotin and the antibody is formed by a publicly known method.

Alternatively, the staining kit B preferably includes, as the reagent, a secondary antibody that has peroxidase and that binds to the primary antibody, a tyramide compound to which the reactive group of the compound (I) is chemically bound, and hydrogen peroxide. The phrase "secondary antibody has peroxidase" means that a peroxidase is bound to the secondary antibody. The form of binding between the peroxidase and the antibody is not particularly limited, and a publicly known conjugation method can be applied. For example, when biotin is bound to the secondary antibody, and avidin or streptavidin is bound to the peroxidase, the form of binding of "secondary antibody-biotin-avidin or streptavidin-peroxidase" is possible.

The staining kit A and the staining kit B may include any primary antibody that binds to the biological sample.

### EXAMPLES

### [Preparation of Reagent]

### <Fluorescent Staining Agent (1)>

In water (RO water) (50 mL) purified by a reverse osmosis membrane treatment, 1 mol/L hydrochloric acid (10 µL) was dissolved. To the resulting solution (about 50 mL), a solution (2.5 µL) in which NKX-4023 (0.01 g) represented by the formula (1) was dissolved in DMSO (0.1 mL) was added to prepare a fluorescent staining agent (1).

### <Fluorescent Staining Agent (2)>

In RO water (50 mL), 1 mol/L hydrochloric acid (10 µL) was dissolved. To the resulting solution (about 50 mL), a solution (25 µL) in which 7-amino-4-methylcoumarin (AMC) (0.013 g) was dissolved in DMSO (0.13 mL) and a solution (0.5 mL) in which 2-picoline borane (0.05 g) was dissolved in DMSO (2 mL) were added to prepare a fluorescent staining agent (2).

### <Fluorescent Staining Agent (3)>

In 212 µL of DMSO, 1 mg of NKX-4190 represented by the formula (4) was dissolved to prepare a dye DMSO solution.

Next, 200 µL of an anti-rabbit IgG antibody PBS buffer solution (2 mg/mL solution, manufacturer: Jackson ImmunoResearch, product number: 111-005-003) was transferred to a centrifugal ultrafiltration filter cartridge (Amicon Ultra-0.5, NMWL: 50K) and concentrated to about 20 µL by centrifuging at 14,000G for five minutes.

The concentrated solution was transferred to a 1.5 mL Eppendorf tube, 0.1 M sodium bicarbonate buffer (pH 8.4) was added thereto to adjust the amount of solution to 200 µL. To the solution, 2 µL of the dye DMSO solution was added, and the resulting solution was incubated at 37°C for 30 minutes to bind NKX-4190 to the antibody.

The reaction solution was transferred to a centrifugal ultrafiltration filter cartridge and concentrated by centrifuging at 14,000G for 10 minutes, 200 µL of a PBS buffer was added thereto, and the resulting solution was again centrifuged at 14,000G for 10 minutes to obtain a concentrated solution. The concentrated solution was charged into a gel filtration column (Micro Bio-Spin Column 6, manufacturer: Bio-Rad, product number: 732-6221) equilibrated with a PBS buffer and eluted with the PBS buffer to remove excessive NKX-4190 that did not bind to the antibody. Thus, an NKX-4190 fluorescent-labeled antibody solution was prepared.

### <Aqueous Osmium Tetroxide Solution>

2% aqueous osmium tetroxide solution (2.5 mL), 0.2 M phosphate buffer (5 mL), and distilled water (2.5 mL) were mixed to prepare 0.5% osmium tetroxide/0.1 M phosphate buffer (10 mL).

### [Example 1]

For a human tissue paraffin-embedded section prepared by a routine method, deparaffinization treatment was performed by a publicly known method including immersing in xylene, ethanol, and ion-exchanged water in this order to prepare a tissue section.

The tissue section was immersed in 0.5% aqueous sodium periodate solution at room temperature for 10 minutes and then washed with water. At least some of hydroxyl groups of a polysaccharide in the tissue section were changed to aldehyde groups by this oxidation treatment.

The tissue section was immersed in 50 mL of the fluorescent staining agent (1) at room temperature for five minutes and then washed with water. A fluorescent-stained sample in which NKX-4023 bound to the polysaccharide, which formed aldehyde groups was obtained by this fluorescence staining.

Next, in a draft chamber, the fluorescent-stained sample was immersed in 10 mL of an aqueous osmium tetroxide solution on ice for 10 minutes to perform postfixation. Subsequently, the fluorescent-stained sample was immersed in ascending series of ethanol at room temperature for three minutes in each ethanol solution to perform dehydration and then immersed in propylene oxide at room temperature for five minutes. Subsequently, the sample was embedded in an epoxy resin, and the resulting fluorescent-stained sample was placed in a thermostatic chamber at 60°C for 24 hours to obtain an embedded sample prepared by curing the epoxy resin.

Next, a section sample thinned to have a thickness of 100 nm was cut out from the embedded sample by a routine method using an ultramicrotome. Lastly, staining was performed with uranyl acetate and a lead stain solution.

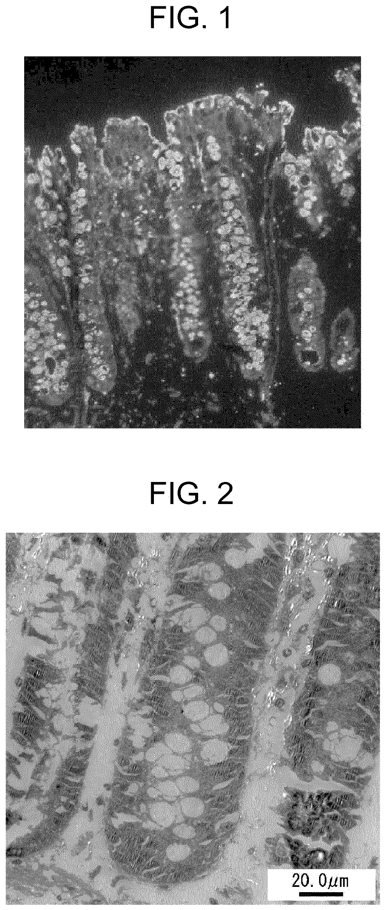

The section sample prepared as described above was observed by the CLEM method, and an image A1 obtained by fluorescence microscopic observation, an image B1 obtained by electron microscopic observation, and an image C1 obtained by superimposing the image A1 and the image B1 are shown in Figs. 1, 2, and 3, respectively.

As shown in the figures, although the above-prepared section sample was subjected to the fixation treatment for electron microscopic observation and other treatment, sufficient fluorescence due to NKX-4023 was detected in mucus, and cells including a sugar chain. This means that NKX-4023 is not subjected to a structural change due to the fixation treatment and other treatment.

Regions where fluorescence was observed by fluorescence microscopic observation were confirmed to be mucus or specific cells by electron microscopic observation, and detailed cellular structures and the like were made clear at a high magnification. These images had high quality to the extent that information useful for, pathological diagnosis can be extracted and the like.

### [Example 2]

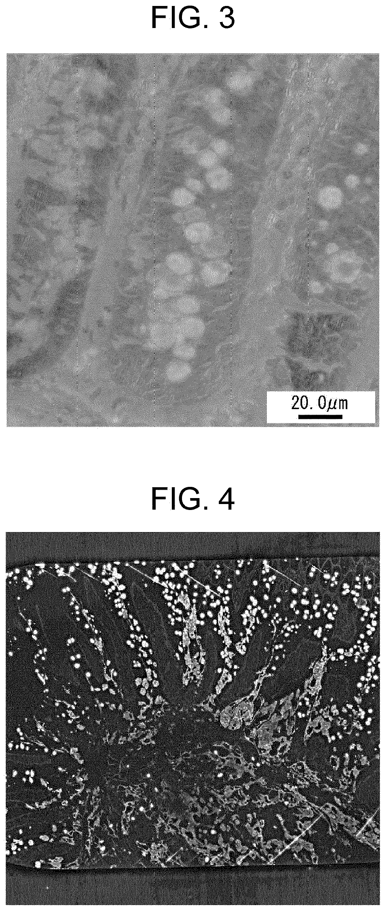

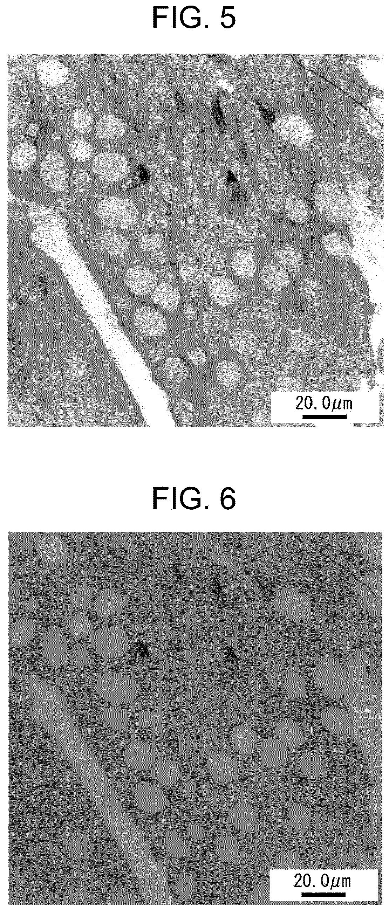

A section sample was obtained as in Example 1 except that a frozen section prepared by immersing tissue in sucrose and then freezing and slicing the tissue was used instead of the paraffin-embedded section, and the fluorescent staining agent (2) was used instead of the fluorescent staining agent (1). This section sample was observed by the CLEM method, and an image A2 obtained by fluorescence microscopic observation, an image B2 obtained by electron microscopic observation, and an image C2 obtained by superimposing the image A2 and the image B2 are shown in Figs. 4, 5, and 6, respectively.

As shown in the figures, images with high quality were obtained as in Example 1.

### [Reference Experiments]

For NKX-4023 represented by the formula (1), AMC represented by the formula (14), and FITC (Fluorescein isothiocyanate isomer-I), which is a well-known fluorescent substance not corresponding to the compound (I), a change in fluorescence intensity before and after contact with an epoxy resin was examined by the following experiments.

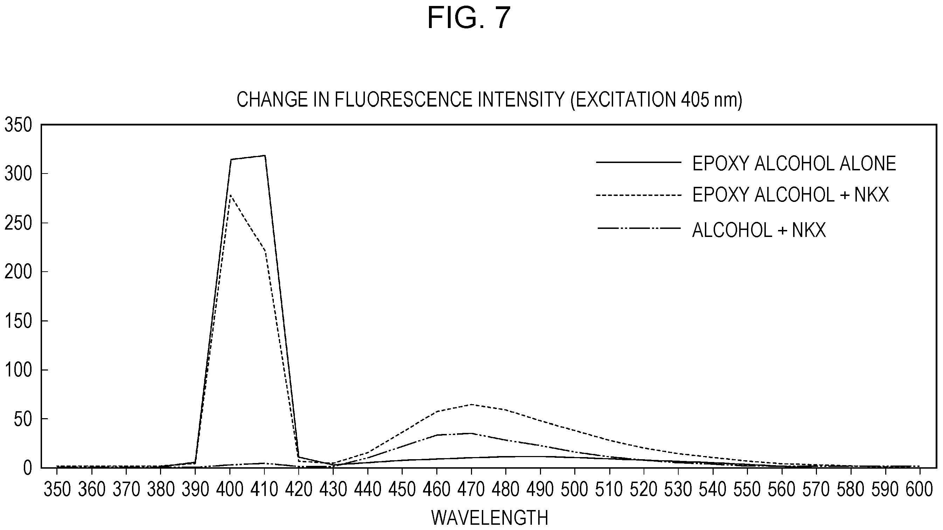

Reference Experiment 1: Into a test tube, 3 mL of a solution prepared by dissolving 0.5 g of propylene oxide, which is an epoxy compound, in 10 mL of alcohol (ethanol) was poured, and a fluorescence spectrum was measured at an excitation wavelength of 405 nm. Subsequently, 3 µL of an NKX-4023 solution (1 mg/mL) was added to the solution in the test tube, and a fluorescence spectrum was measured in the same manner. According to the results, high-intensity fluorescence having a peak at around 465 nm was observed (refer to Fig. 7).

Into another test tube, 3 mL of alcohol (ethanol) and 3 µL of an NKX-4023 solution (1 mg/mL) were poured, and a fluorescence spectrum was measured at an excitation wavelength of 405 nm. According to the results, high-intensity fluorescence having a peak at around 465 nm was observed (refer to Fig. 7).

The above results demonstrate that NKX-4023 has a sufficient fluorescence intensity even after coming in contact with the epoxy compound, and coincide with the results of Example 1. Accordingly, it is considered that coumarin fluorescent dyes that do not lose fluorescence in similar reference experiments, can be used as the compound (I) of the present invention.

In the graph of Fig. 7, the solid line represents the measurement results of the solution in which propylene oxide was dissolved in alcohol (ethanol). The broken line represents the measurement results of the solution in which NKX-4023 was dissolved in alcohol (ethanol) containing propylene oxide. The two-dot chain line represents the measurement results of the solution in which NKX-4023 was dissolved in alcohol (ethanol). The vertical axis represents the fluorescence intensity (arbitrary unit), and the horizontal axis represents the fluorescence wavelength (nm).

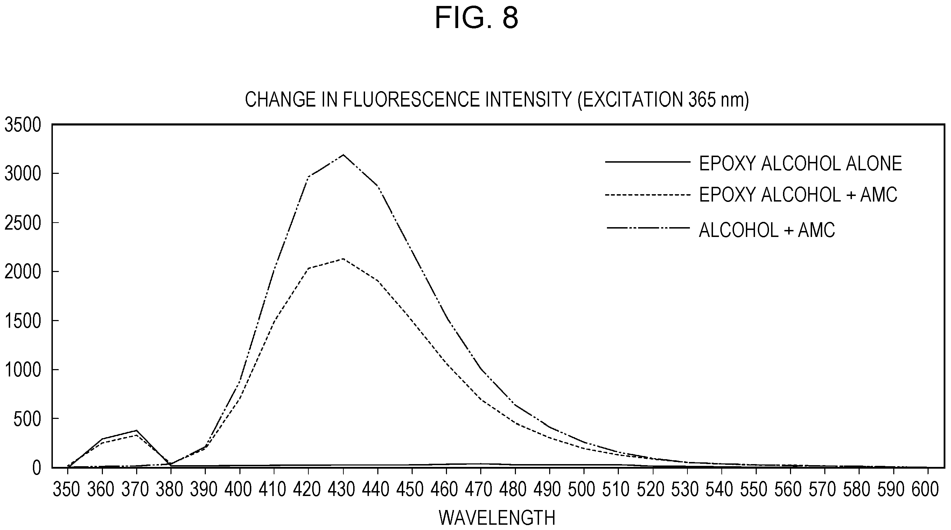

Reference Experiment 2: Into a test tube, 3 mL of a solution prepared by dissolving 0.5 g of propylene oxide, which is an epoxy compound, in 10 mL of alcohol (ethanol) was poured, and a fluorescence spectrum was measured at an excitation wavelength of 365 nm. Subsequently, 3 µL of an AMC solution (1 mg/mL) was added to the solution in the test tube, and a fluorescence spectrum was measured in the same manner. According to the results, high-intensity fluorescence having a peak at around 430 nm was observed (refer to Fig. 8).

Into another test tube, 3 mL of alcohol (ethanol) and 3 µL of an AMC solution (1 mg/mL) were poured, and a fluorescence spectrum was measured at an excitation wavelength of 365 nm. According to the results, high-intensity fluorescence having a peak at around 430 nm was observed (refer to Fig. 8).

The above results demonstrate that AMC has a sufficient fluorescence intensity even after coming in contact with the epoxy compound, and coincide with the results of Example 2. Accordingly, it is considered that coumarin fluorescent compounds that do not lose fluorescence in similar reference experiments, can be used as the compound (I) of the present invention.

In the graph of Fig. 8, the solid line represents the measurement results of the solution in which propylene oxide was dissolved in alcohol (ethanol). The broken line represents the measurement results of the solution in which AMC was dissolved in alcohol (ethanol) containing propylene oxide. The two-dot chain line represents the measurement results of the solution in which AMC was dissolved in alcohol (ethanol). The vertical axis represents the fluorescence intensity (arbitrary unit), and the horizontal axis represents the fluorescence wavelength (nm).

Reference Experiment 3: Into a test tube, 3 mL of a solution prepared by dissolving 0.5 g of propylene oxide, which is an epoxy compound, in 10 mL of alcohol (ethanol) was poured, and a fluorescence spectrum was measured at an excitation wavelength of 488 nm. Subsequently, 3 µL of an FITC solution (1 mg/mL) was added to the solution in the test tube, and a fluorescence spectrum was measured in the same manner.

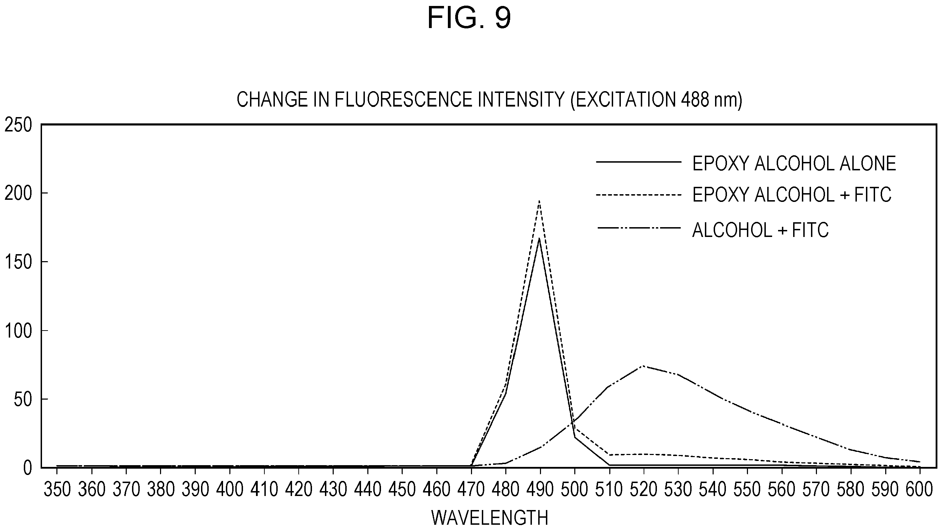

According to the results, no clear peak of fluorescence due to FITC was observed in a range of 350 nm to 600 nm (refer to Fig. 9).

Into another test tube, 3 mL of alcohol (ethanol) and 3 µL of an FITC solution (1 mg/mL) were poured, and a fluorescence spectrum was measured at an excitation wavelength of 488 nm. According to the results, fluorescence having a sufficient intensity and having a peak at around 520 nm was observed (refer to Fig. 9).

In the graph of Fig. 9, the solid line represents the measurement results of the solution in which propylene oxide was dissolved in alcohol (ethanol). The broken line represents the measurement results of the solution in which FITC was dissolved in alcohol (ethanol) containing propylene oxide. The two-dot chain line represents the measurement results of the solution in which FITC was dissolved in alcohol (ethanol). The vertical axis represents the fluorescence intensity (arbitrary unit), and the horizontal axis represents the fluorescence wavelength (nm).

The above results demonstrated that the coumarin fluorescent dyes each corresponding to the compound (I) are not subjected to a structural change by the epoxy compound and that FITC that does not correspond to the compound (I) is subjected to a structural change (or decomposed) by the epoxy compound, and the fluorescence is deactivated.

Reference Experiment 4: Into a test tube, 3 mL of a solution prepared by dissolving 0.5 g of propylene oxide in 10 mL of ethanol was poured, and a fluorescence spectrum was measured at an excitation wavelength of 324 nm. Subsequently, 3 µL of a solution (1 mg/mL) of the reference compound 101 represented by the formula (101) was added to the solution in the test tube, and a fluorescence spectrum was measured in the same manner.

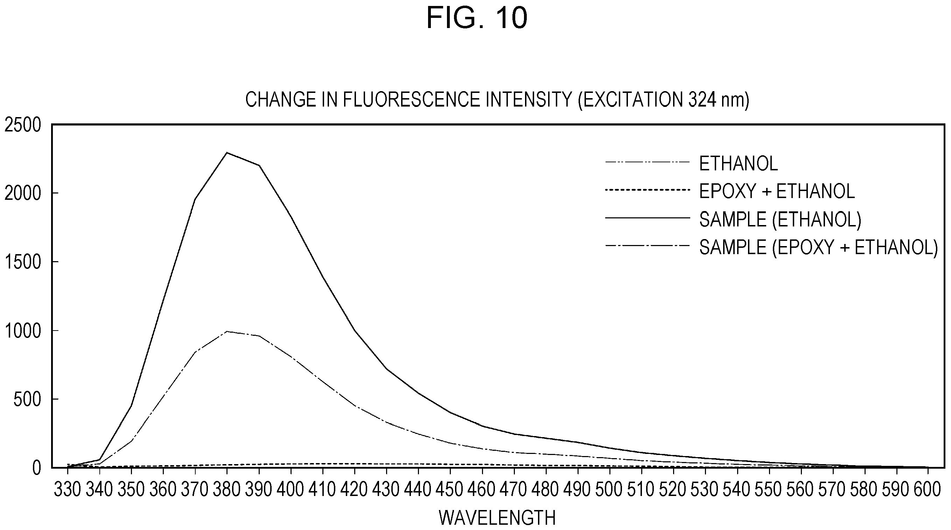

According to the results, fluorescence having a peak at around 380 nm was observed (refer to Fig. 10).

Into another test tube, 3 mL of ethanol and 3 µL of a solution (1 mg/mL) of the reference compound 101 were poured, and a fluorescence spectrum was measured at an excitation wavelength of 324 nm. According to the results, high-intensity fluorescence having a peak at around 380 nm was observed (refer to Fig. 10).

The above results demonstrate that the reference compound 101 was subjected to a certain fluorescence-intensity reduction effect by contact with the epoxy compound, but the loss of fluorescence intensity did not occur. Accordingly, the reference compound 101 can be used as the compound (I) of the present invention if the reactive group is introduced therein.

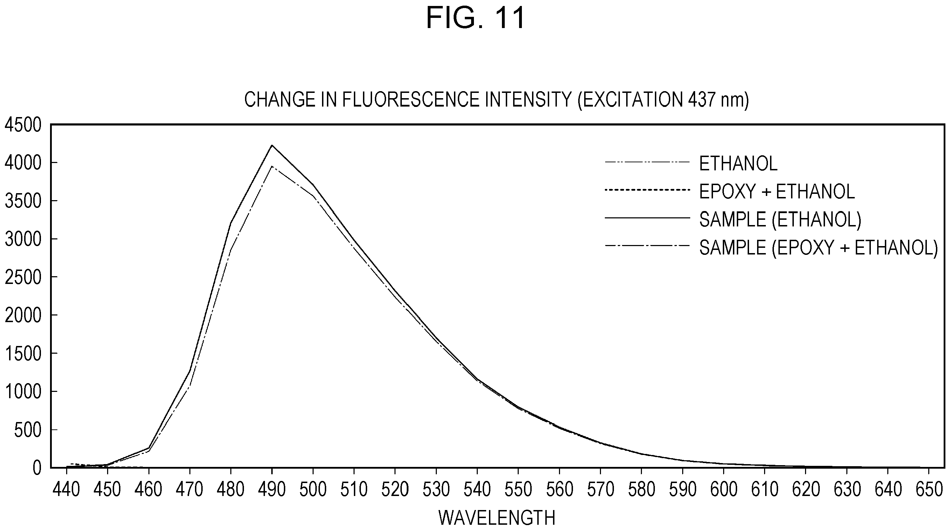

In the graph of Fig. 10, the two-dot chain line represents the measurement results of ethanol alone. The broken line represents the measurement results of the solution in which propylene oxide, which is an epoxy compound, was dissolved in ethanol. The solid line represents the measurement results of the solution in which the reference compound serving as a sample was dissolved in ethanol. The one-dot chain line represents the measurement results of the solution in which the reference compound serving as a sample was dissolved in ethanol containing propylene oxide. The vertical axis represents the fluorescence intensity (arbitrary unit), and the horizontal axis represents the fluorescence wavelength (nm). Figs. 11 to 20 also show measurement results in the same manner.

Reference Experiment 5: Into a test tube, 3 mL of a solution prepared by dissolving 0.5 g of propylene oxide in 10 mL of ethanol was poured, and a fluorescence spectrum was measured at an excitation wavelength of 437 nm. Subsequently, 3 µL of a solution (1 mg/mL) of the reference compound 102 represented by the formula (102) was added to the solution in the test tube, and a fluorescence spectrum was measured in the same manner.

According to the results, fluorescence having a peak at around 490 nm was observed (refer to Fig. 11).

Into another test tube, 3 mL of ethanol and 3 µL of a solution (1 mg/mL) of the reference compound 102 were poured, and a fluorescence spectrum was measured at an excitation wavelength of 437 nm. According to the results, fluorescence having a peak at around 490 nm was observed (refer to Fig. 11).

The above results demonstrate that the reference compound 102 has a strong fluorescence intensity even after coming in contact with the epoxy compound. Accordingly, the reference compound 102 can be used as the compound (I) of the present invention if the reactive group is introduced therein.

Reference Experiment 6: Into a test tube, 3 mL of a solution prepared by dissolving 0.5 g of propylene oxide in 10 mL of ethanol was poured, and a fluorescence spectrum was measured at an excitation wavelength of 460 nm. Subsequently, 3 µL of a solution (1 mg/mL) of the reference compound 103 represented by the formula (103) was added to the solution in the test tube, and a fluorescence spectrum was measured in the same manner.

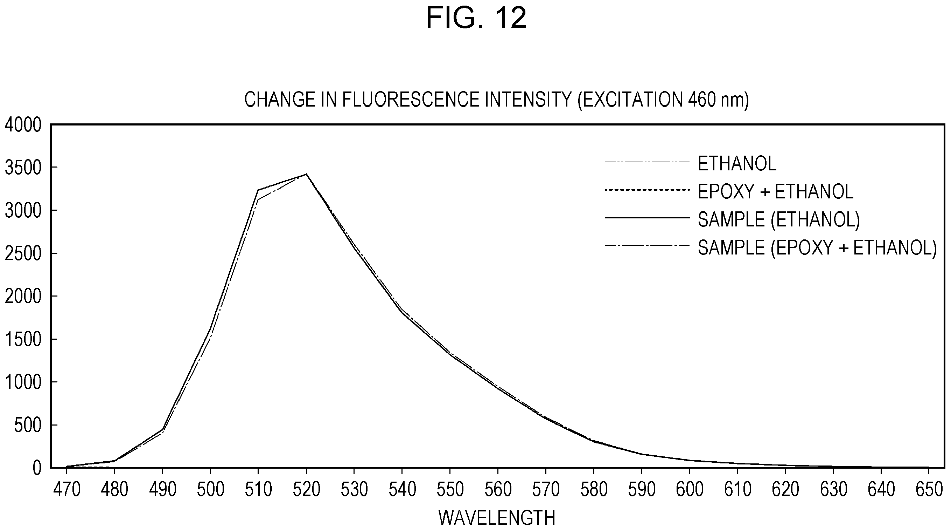

According to the results, fluorescence having a peak at around 520 nm was observed (refer to Fig. 12).

Into another test tube, 3 mL of ethanol and 3 µL of a solution (1 mg/mL) of the reference compound 103 were poured, and a fluorescence spectrum was measured at an excitation wavelength of 460 nm. According to the results, fluorescence having a peak at around 520 nm was observed (refer to Fig. 12).

The above results demonstrate that the reference compound 103 has a strong fluorescence intensity even after coming in contact with the epoxy compound. Accordingly, the reference compound 103 can be used as the compound (I) of the present invention if the reactive group is introduced therein.

Reference Experiment 7: Into a test tube, 3 mL of a solution prepared by dissolving 0.5 g of propylene oxide in 10 mL of ethanol was poured, and a fluorescence spectrum was measured at an excitation wavelength of 459 nm. Subsequently, 3 µL of a solution (1 mg/mL) of the reference compound 104 represented by the formula (104) was added to the solution in the test tube, and a fluorescence spectrum was measured in the same manner.

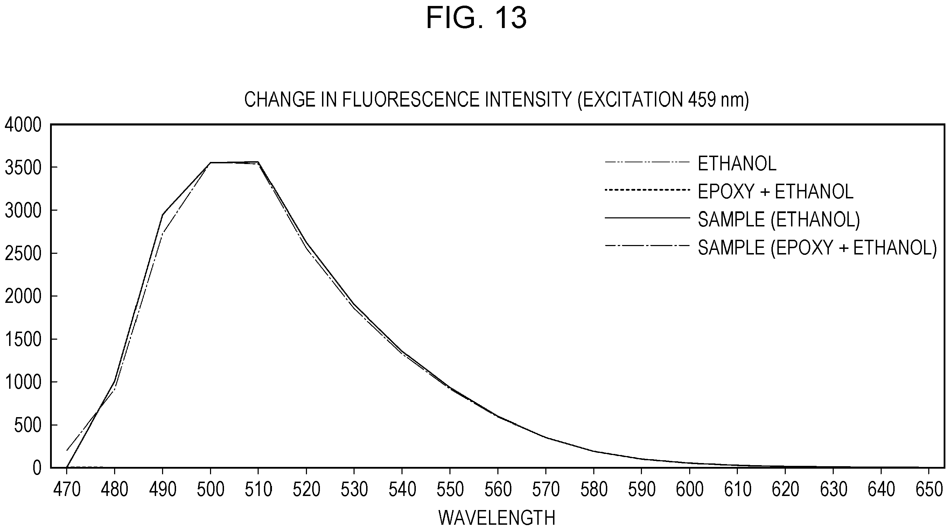

According to the results, fluorescence having a peak at around 505 nm was observed (refer to Fig. 13).

Into another test tube, 3 mL of ethanol and 3 µL of a solution (1 mg/mL) of the reference compound 104 were poured, and a fluorescence spectrum was measured at an excitation wavelength of 459 nm. According to the results, fluorescence having a peak at around 505 nm was observed (refer to Fig. 13).

The above results demonstrate that the reference compound 104 has a strong fluorescence intensity even after coming in contact with the epoxy compound. Accordingly, the reference compound 104 can be used as the compound (I) of the present invention if the reactive group is introduced therein.

Reference Experiment 8: Into a test tube, 3 mL of a solution prepared by dissolving 0.5 g of propylene oxide in 10 mL of ethanol was poured, and a fluorescence spectrum was measured at an excitation wavelength of 387 nm. Subsequently, 3 µL of a solution (1 mg/mL) of the reference compound 105 represented by the formula (105) was added to the solution in the test tube, and a fluorescence spectrum was measured in the same manner.

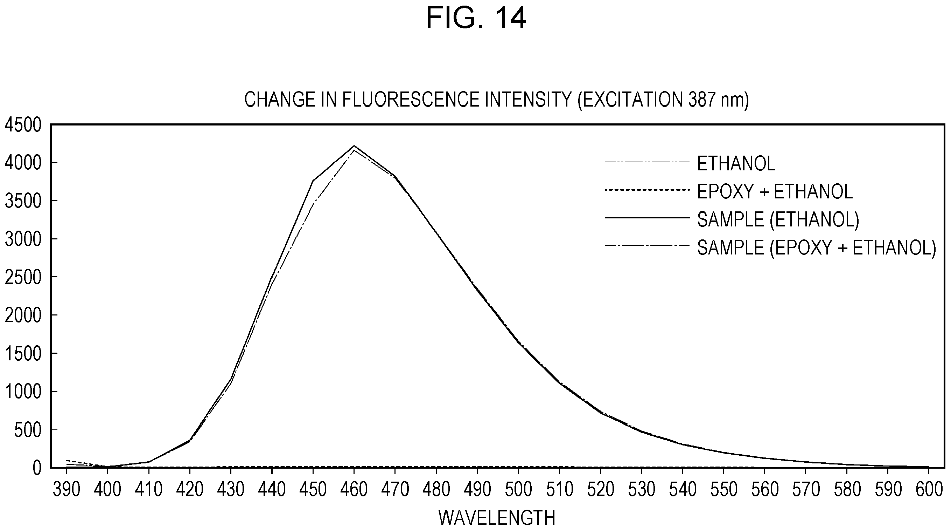

According to the results, fluorescence having a peak at around 460 nm was observed (refer to Fig. 14).

Into another test tube, 3 mL of ethanol and 3 µL of a solution (1 mg/mL) of the reference compound 105 were poured, and a fluorescence spectrum was measured at an excitation wavelength of 387 nm. According to the results, fluorescence having a peak at around 460 nm was observed (refer to Fig. 14).

The above results demonstrate that the reference compound 105 has a strong fluorescence intensity even after coming in contact with the epoxy compound. Accordingly, the reference compound 105 can be used as the compound (I) of the present invention if the reactive group is introduced therein.

Reference Experiment 9: Into a test tube, 3 mL of a solution prepared by dissolving 0.5 g of propylene oxide in 10 mL of ethanol was poured, and a fluorescence spectrum was measured at an excitation wavelength of 457 nm. Subsequently, 3 µL of a solution (1 mg/mL) of the reference compound 106 represented by the formula (106) was added to the solution in the test tube, and a fluorescence spectrum was measured in the same manner.

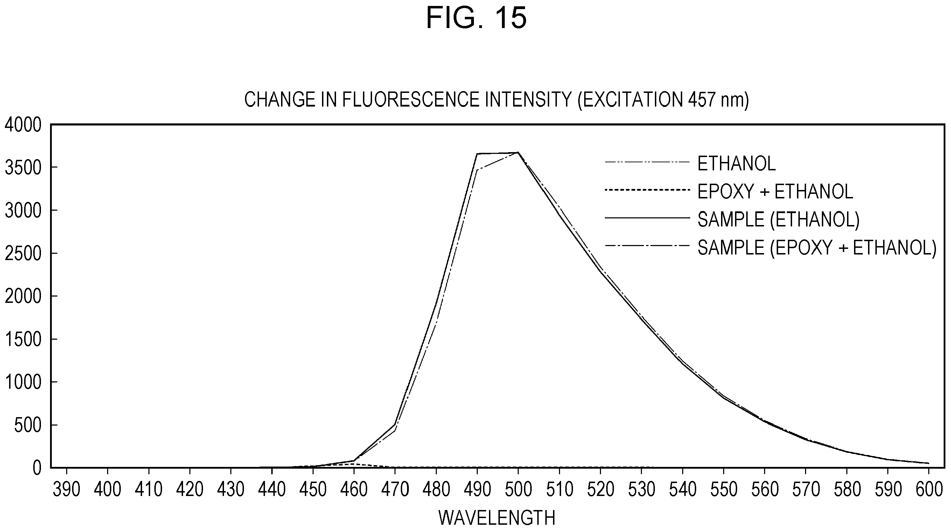

According to the results, fluorescence having a peak at around 500 nm was observed (refer to Fig. 15).

Into another test tube, 3 mL of ethanol and 3 µL of a solution (1 mg/mL) of the reference compound 106 were poured, and a fluorescence spectrum was measured at an excitation wavelength of 457 nm. According to the results, fluorescence having a peak at around 500 nm was observed (refer to Fig. 15).

The above results demonstrate that the reference compound 106 has a strong fluorescence intensity even after coming in contact with the epoxy compound. Accordingly, the reference compound 106 can be used as the compound (I) of the present invention if the reactive group is introduced therein.

Reference Experiment 10: Into a test tube, 3 mL of a solution prepared by dissolving 0.5 g of propylene oxide in 10 mL of ethanol was poured, and a fluorescence spectrum was measured at an excitation wavelength of 455 nm. Subsequently, 3 µL of a solution (1 mg/mL) of the reference compound 107 represented by the formula (107) was added to the solution in the test tube, and a fluorescence spectrum was measured in the same manner.

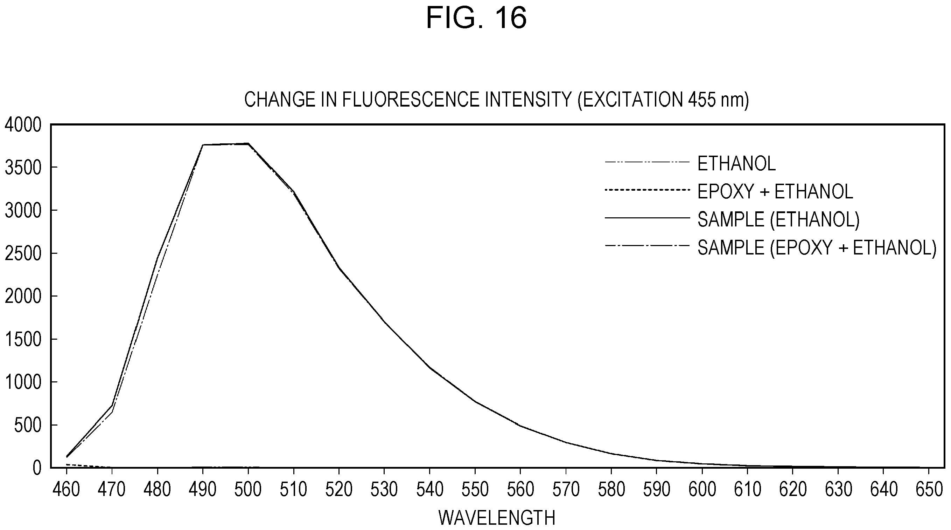

According to the results, fluorescence having a peak at around 495 nm was observed (refer to Fig. 16).

Into another test tube, 3 mL of ethanol and 3 µL of a solution (1 mg/mL) of the reference compound 107 were poured, and a fluorescence spectrum was measured at an excitation wavelength of 455 nm. According to the results, fluorescence having a peak at around 495 nm was observed (refer to Fig. 16) .

The above results demonstrate that the reference compound 107 has a strong fluorescence intensity even after coming in contact with the epoxy compound. Accordingly, the reference compound 107 can be used as the compound (I) of the present invention if the reactive group is introduced therein.

Reference Experiment 11: Into a test tube, 3 mL of a solution prepared by dissolving 0.5 g of propylene oxide in 10 mL of ethanol was poured, and a fluorescence spectrum was measured at an excitation wavelength of 459 nm. Subsequently, 3 µL of a solution (1 mg/mL) of the reference compound 108 represented by the formula (108) was added to the solution in the test tube, and a fluorescence spectrum was measured in the same manner.

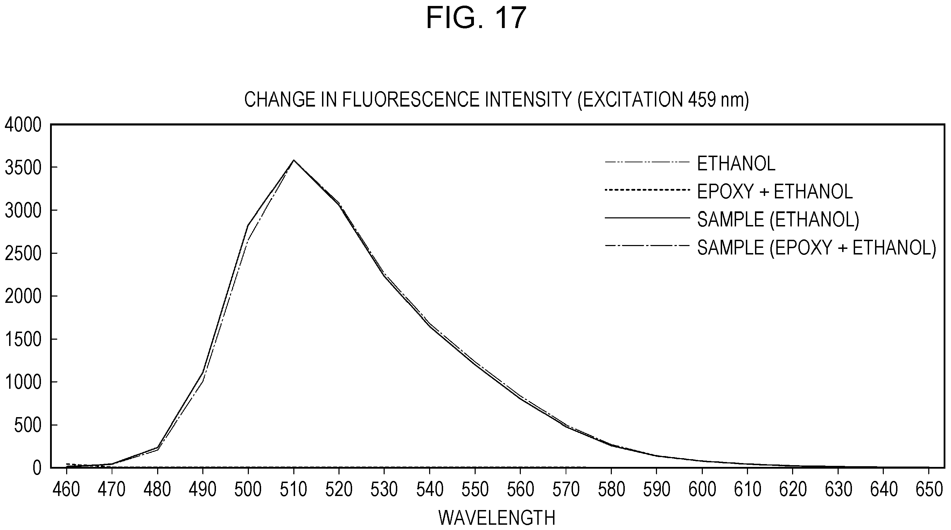

According to the results, fluorescence having a peak at around 510 nm was observed (refer to Fig. 17).

Into another test tube, 3 mL of ethanol and 3 µL of a solution (1 mg/mL) of the reference compound 108 were poured, and a fluorescence spectrum was measured at an excitation wavelength of 459 nm. According to the results, fluorescence having a peak at around 510 nm was observed (refer to Fig. 17).

The above results demonstrate that the reference compound 108 has a strong fluorescence intensity even after coming in contact with the epoxy compound. Accordingly, the reference compound 108 can be used as the compound (I) of the present invention if the reactive group is introduced therein.

Reference Experiment 12: Into a test tube, 3 mL of a solution prepared by dissolving 0.5 g of propylene oxide in 10 mL of ethanol was poured, and a fluorescence spectrum was measured at an excitation wavelength of 435 nm. Subsequently, 3 µL of a solution (1 mg/mL) of the reference compound 109 represented by the formula (109) was added to the solution in the test tube, and a fluorescence spectrum was measured in the same manner.

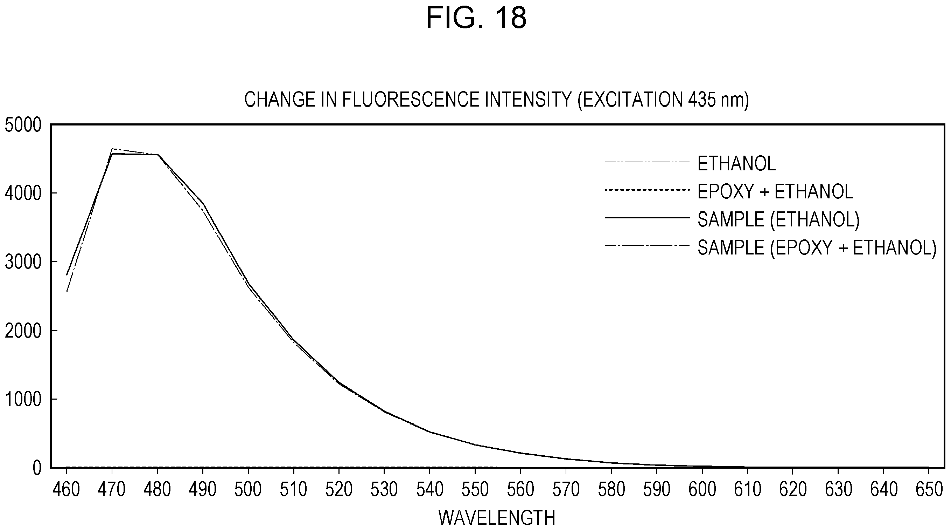

According to the results, fluorescence having a peak at around 475 nm was observed (refer to Fig. 18).

Into another test tube, 3 mL of ethanol and 3 µL of a solution (1 mg/mL) of the reference compound 109 were poured, and a fluorescence spectrum was measured at an excitation wavelength of 435 nm. According to the results, fluorescence having a peak at around 475 nm was observed (refer to Fig. 18).

The above results demonstrate that the reference compound 109 has a strong fluorescence intensity even after coming in contact with the epoxy compound. Accordingly, the reference compound 109 can be used as the compound (I) of the present invention if the reactive group is introduced therein.

Reference Experiment 13: Into a test tube, 3 mL of a solution prepared by dissolving 0.5 g of propylene oxide in 10 mL of ethanol was poured, and a fluorescence spectrum was measured at an excitation wavelength of 377 nm. Subsequently, 3 µL of a solution (1 mg/mL) of the reference compound 111 represented by the formula (111) was added to the solution in the test tube, and a fluorescence spectrum was measured in the same manner.

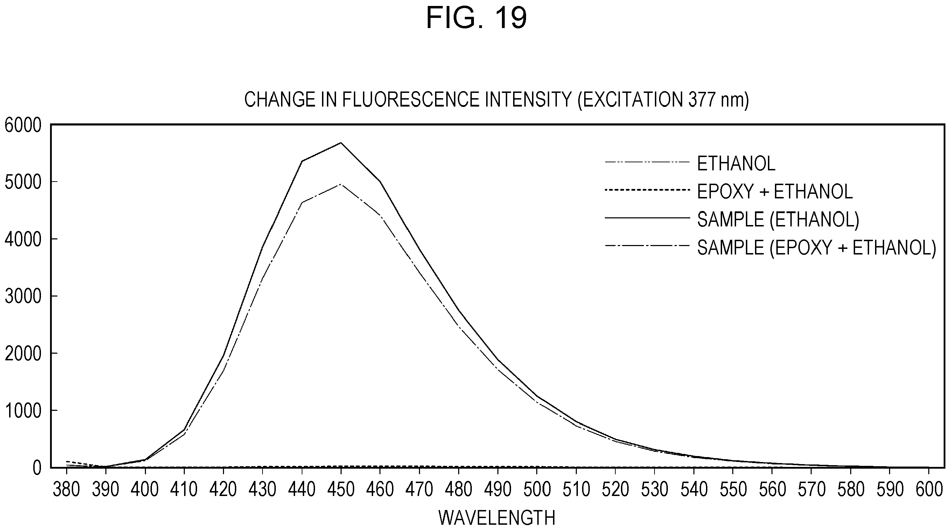

According to the results, fluorescence having a peak at around 450 nm was observed (refer to Fig. 19).

Into another test tube, 3 mL of ethanol and 3 µL of a solution (1 mg/mL) of the reference compound 111 were poured, and a fluorescence spectrum was measured at an excitation wavelength of 377 nm. According to the results, fluorescence having a peak at around 450 nm was observed (refer to Fig. 19) .

The above results demonstrate that the reference compound 111 has a strong fluorescence intensity even after coming in contact with the epoxy compound. Accordingly, the reference compound 111 can be used as the compound (I) of the present invention if the reactive group is introduced therein.

Reference Experiment 14: Into a test tube, 3 mL of a solution prepared by dissolving 0.5 g of propylene oxide in 10 mL of ethanol was poured, and a fluorescence spectrum was measured at an excitation wavelength of 528 nm. Subsequently, 3 µL of a solution (1 mg/mL) of the reference compound 112 represented by the formula (112) was added to the solution in the test tube, and a fluorescence spectrum was measured in the same manner.

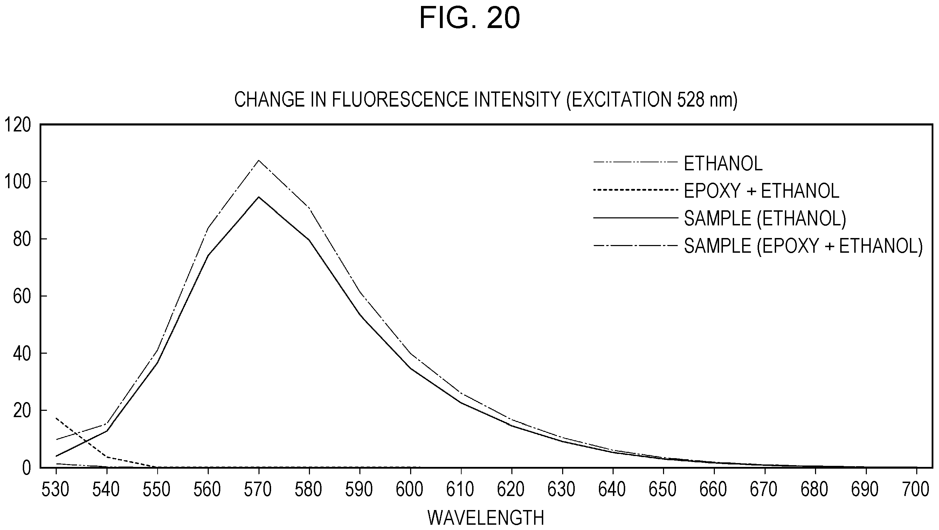

According to the results, fluorescence having a peak at around 570 nm was observed (refer to Fig. 20).

Into another test tube, 3 mL of ethanol and 3 µL of a solution (1 mg/mL) of the reference compound 112 were poured, and a fluorescence spectrum was measured at an excitation wavelength of 528 nm. According to the results, fluorescence having a peak at around 570 nm was observed (refer to Fig. 20).

The above results demonstrate that the reference compound 112 has a strong fluorescence intensity even after coming in contact with the epoxy compound. Accordingly, the reference compound 112 can be used as the compound (I) of the present invention if the reactive group is introduced therein.

The reference experiments described above are each a useful method for simply examining, when a section sample provided for electron microscopic observation is embedded in an epoxy resin, whether a fluorescent dye contained in the section sample is subjected to a structural change by an epoxy compound. If a fluorescent dye maintains the structure that emits fluorescence after the fluorescent dye is brought into contact with an epoxy compound in a test tube, the fluorescent dye is considered to have resistance to epoxy resin embedding treatment in a section sample provided for electron microscopic observation. That is, the fluorescent dye having the resistance is a preferred fluorescent dye that can be used in the microscopic observation method according to the present invention.

### <Preparation of Section Sample of Skin Tissue>

Two paraffin-embedded sections of human skin tissue were prepared by a routine method. The two skin sections were cut out from substantially the same position in the skin tissue.

As described in detail later, one skin section was subjected to immunofluorescence staining using a labeled antibody solution of the fluorescent staining agent (3). The other skin section was subjected to DAB staining using an antibody labeled with a commercially available peroxidase enzyme.

### [Example 3]

### (Immunofluorescence Staining)

For a paraffin-embedded section of a human skin tissue sample prepared by a routine method, deparaffinization treatment was performed by a publicly known method including immersing in xylene, ethanol, and ion-exchanged water in this order to prepare a tissue section. Furthermore, the tissue section was immersed in a 10 mM citric acid buffer (pH 6.0) heated at 95°C in a hot bath, incubated for 45 minutes, and allowed to stand at room temperature for 30 minutes to perform antigen retrieval treatment.

In order to prevent a nonspecific reaction in the tissue, the tissue section was immersed, at room temperature for 30 minutes, in a blocking reagent prepared by dissolving 4 g of a blocking agent (manufacturer: DS Pharma Biomedical Co., Ltd., product number: UK-B80) in 100 mL of a PBS buffer.

A primary antibody solution was prepared by diluting an anti-cytokeratin antibody solution (manufacturer: DAKO, product number: Z0622) 500-fold with the above blocking reagent. The tissue section that had been subjected to the blocking treatment was immersed in this solution at 4°C overnight to react with the primary antibody, and the tissue section was then washed with PBS buffer. A secondary antibody solution was prepared by diluting the labeled antibody solution of the fluorescent staining agent (3) 100-fold with the above blocking reagent. The tissue section was immersed in this solution at room temperature for four hours to react with the secondary antibody, and the tissue section was then washed with PBS buffer to prepare an immunofluorescent-stained sample.

Next, in a draft chamber, the immunofluorescent-stained sample was immersed in 10 mL of 0.5% aqueous glutaraldehyde solution at room temperature for five minutes, washed with PBS buffer, subsequently immersed in 10 mL of 0.5% aqueous osmium tetroxide solution at room temperature for two minutes, and washed with water. The immunofluorescent-stained sample that had been subjected to a series of this treatment (postfixation) was immersed in ascending series of ethanol at room temperature for three minutes in each ethanol solution to perform dehydration. Subsequently, the dehydrated immunofluorescent-stained sample was immersed in propylene oxide at room temperature for five minutes, and the immunofluorescent-stained sample impregnated with propylene oxide was placed in a thermostatic chamber at 60°C for 24 hours to obtain a sample (embedded sample) embedded in an epoxy resin formed by curing propylene oxide.

Next, a section sample thinned to have a thickness of 500 nm was cut out from the embedded sample by a routine method using an ultramicrotome.

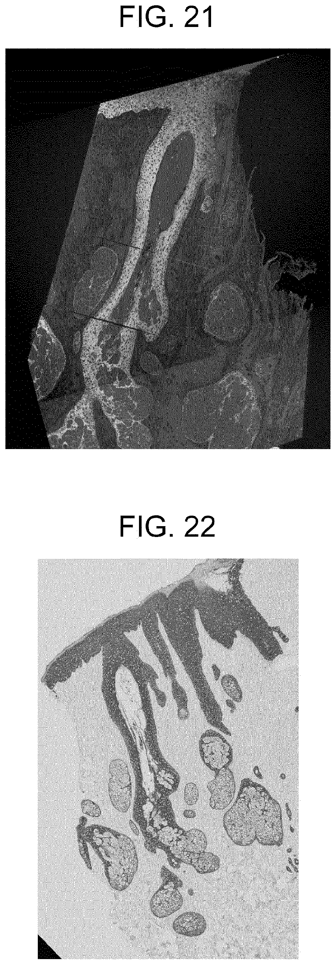

An image A3 obtained by observing this section sample with a fluorescence microscope is shown in Fig. 21. In this case, the observation was performed at an excitation wavelength of 385 nm using a filter or the like used for fluorescence observation of DAPI, which is a publicly known dye. According to the results, strong blue fluorescence was shown in regions where keratin was considered to be present and the morphology of the tissue was highlighted. The blue fluorescence is derived from NKX-4190 bound to the labeled antibody.

An ultrathin section cut out to have a thickness of 100 nm was observed with a fluorescence microscope in the same manner as described above. Although not shown in the figure, blue fluorescence was shown in regions where keratin was considered to be present, and the morphology of the tissue could be confirmed.

### [Comparative Example 1]

### <DAB Staining>

DAB staining as described above was performed using the other skin section in accordance with a routine method with an automatic immunostaining device (manufactured by Leica Biosystems: BOND MAX). In this case, a color development kit (BOND Polymer Refine Detection, product code: DS9800) was used. In summary, after reacting an anti-keratin antibody to the other skin section, an enzyme-labeled antibody that binds to the anti-keratin antibody is reacted as a secondary antibody. When DAB, which is a substrate of the enzyme, is brought into contact with the section sample obtained as described above, regions where the anti-keratin antibody and the enzyme-labeled antibody are present are stained brown.

An image B3 obtained by observing the section sample subjected to DAB staining with a microscope is shown in Fig. 22. It is publicly known that the anti-keratin antibody specifically binds to keratin, and thus it is obvious that regions where keratinocytes (cornified cells having keratin) are present are stained brown.

### <Superimposition of Immunofluorescence Staining and DAB Staining>

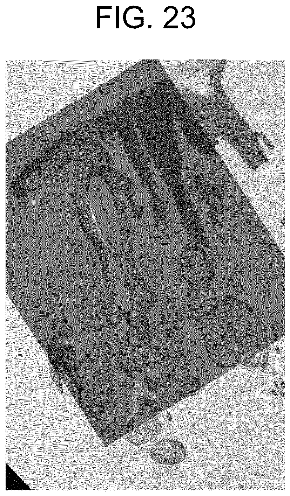

Fig. 23 shows the results of an image C3 obtained by superimposing the fluorescence observation image A3 shown in Fig. 21 and the observation image B3 of DAB staining shown in Fig. 22. In this image C3, regions where fluorescence is observed and regions where brown is observed match very well. Accordingly, it was confirmed that, in the section sample of the skin tissue, the secondary antibody labeled with NKX-4190 binds to the anti-keratin antibody, thereby showing, by fluorescence, the regions where keratinocytes are present.

In addition, since the section sample used in this fluorescence observation has already been subjected to preliminary treatment (fixation with glutaraldehyde, postfixation with osmium tetroxide, and embedding in an epoxy resin) for performing electron microscopic observation, the section sample can be observed with an electron microscope without performing another preliminary treatment.

It was also confirmed that NKX-4190 is not subjected to a structural change even after the preliminary treatment for performing electron microscopic observation, and the fluorescence is not deactivated.

The above results are confirmed that an antibody labeled with the compound (I) is useful for immunofluorescence staining and that fluorescence of the compound (I) is not deactivated by the preliminary treatment for performing electron microscopic observation wherein it is obvious that the compound (I) is useful as a dye used in the CLEM method.

The individual configurations, combinations thereof, and other details of the individual embodiments described above are merely illustrative, and additions, omissions, substitutions, and other modifications of publicly known configurations can be made without departing from the spirit of the present invention.

### Industrial Applicability

The present invention is widely applicable to technical fields in which cells or tissue sections are stained, for example, medical science and medical fields such as pathological diagnosis and studies on cytology and histology.

## Claims

1. A staining method comprising:
staining a biological sample with a coumarin fluorescent dye represented by formula (I) below to provide a fluorescent-stained sample; and
bringing the fluorescent-stained sample into contact with osmium tetroxide, further embedding the sample in an epoxy resin, and subsequently slicing the sample to provide a section sample including the fluorescent-stained sample: 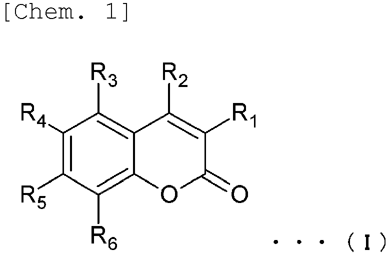 [wherein R₁ to R₆ are each independently a hydrogen atom or any substituent,
at least one selected from R₁, R₂, and R₅ includes a reactive group,
the reactive group is a functional group capable of forming a chemical bond with an aldehyde, an amine, or a thiol,
R₁ may have an aromatic ring, and one or more hydrogen atoms bound to the aromatic ring may be substituted with the reactive group through a linker,
when the aromatic ring is an aromatic heterocycle having a heteroatom other than a carbon atom, the reactive group may be bound to the heteroatom through a linker, and
R₄ and R₆ may each independently form a ring together with R₅] .

2. The staining method according to Claim 1, comprising oxidation treatment, wherein the biological sample is subjected to oxidation treatment in advance to change at least some of hydroxyl groups of a polysaccharide contained in the biological sample to aldehyde groups.

3. The staining method according to Claim 2, comprising binding the coumarin fluorescent dye to some of the aldehyde groups formed in the polysaccharide to provide the fluorescent-stained sample.

4. The staining method according to Claim 1, comprising binding a primary antibody having the coumarin fluorescent dye to the biological sample.

5. The staining method according to Claim 1, comprising binding a primary antibody to the biological sample, and subsequently indirectly binding the coumarin fluorescent dye to the primary antibody.

6. The staining method according to Claim 5,
wherein the primary antibody has biotin, and
avidin or streptavidin having a plurality of the coumarin fluorescent dyes is bound to the biotin.

7. The staining method according to Claim 1, comprising binding a primary antibody or secondary antibody having a peroxidase to the biological sample, converting a tyramide compound having the coumarin fluorescent dye into a radical by a catalytic action of the peroxidase in the presence of hydrogen peroxide, and binding the tyramide compound to a protein present in the vicinity of the primary antibody or the secondary antibody to provide the fluorescent-stained sample.

8. A microscopic observation method comprising observing a section sample obtained by the staining method according to any one of Claims 1 to 7 with both a fluorescence microscope and an electron microscope.

9. The microscopic observation method according to Claim 8, comprising superimposing an image obtained by observation with the fluorescence microscope and an image obtained by observation with the electron microscope to obtain an image.

10. A staining agent comprising a coumarin fluorescent dye represented by formula (I) below, the staining agent being used in an application for staining a biological sample provided for electron microscopic observation: 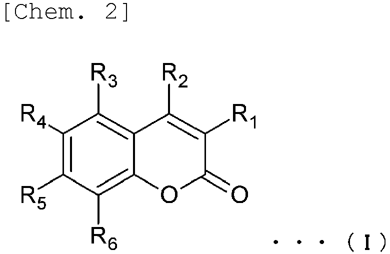 [wherein R₁ to R₆ are each independently a hydrogen atom or any substituent,
at least one selected from R₁, R₂, and R₅ includes a reactive group,
the reactive group is a functional group capable of forming a chemical bond with an aldehyde, an amine, or a thiol,
R₁ may have an aromatic ring, and one or more hydrogen atoms bound to the aromatic ring may be substituted with the reactive group through a linker,
when the aromatic ring is an aromatic heterocycle having a heteroatom other than a carbon atom, the reactive group may be bound to the heteroatom through a linker, and
R₄ and R₆ may each independently form a ring together with R₅] .

11. The staining agent according to Claim 10, wherein the biological sample is also provided for fluorescence microscopic observation after being stained.

12. A staining agent comprising a labeled substance to which a reactive group of a coumarin fluorescent dye represented by formula (I) below is chemically bound, the staining agent being used in an application for staining a biological sample provided for electron microscopic observation: 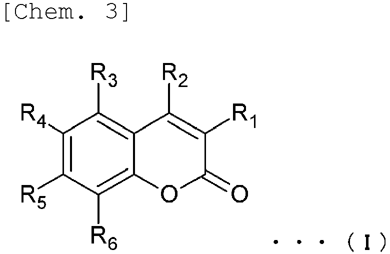 [wherein R₁ to R₆ are each independently a hydrogen atom or any substituent,
at least one selected from R₁, R₂, and R₅ includes a reactive group,
the reactive group is a functional group capable of forming a chemical bond with an aldehyde, an amine, or a thiol,
R₁ may have an aromatic ring, and one or more hydrogen atoms bound to the aromatic ring may be substituted with the reactive group through a linker,
when the aromatic ring is an aromatic heterocycle having a heteroatom other than a carbon atom, the reactive group may be bound to the heteroatom through a linker, and
R₄ and R₆ may each independently form a ring together with R₅] .

13. The staining agent according to Claim 12, wherein the labeled substance is at least one selected from the group consisting of an antibody, avidin, streptavidin, and a tyramide compound.

14. The staining agent according to Claim 12 or 13, wherein the biological sample is also provided for fluorescence microscopic observation after being stained.

15. A staining kit comprising the staining agent according to any one of Claims 10 to 14, the staining kit being used in an application for staining a biological sample provided for electron microscopic observation.

16. The staining kit according to Claim 15, comprising the staining agent containing at least one labeled substance according to any one of Claims 12 to 14; and at least one reagent that indirectly binds the labeled substance to any primary antibody that binds to the biological sample.

17. The staining kit according to Claim 16, comprising:
as the reagent, a secondary antibody that has biotin and that binds to the primary antibody; and
as the labeled substance, avidin or streptavidin to which the reactive group of the coumarin fluorescent dye is chemically bound.

18. The staining kit according to Claim 16, comprising:
as the reagent, a secondary antibody that has a peroxidase and that binds to the primary antibody; and
as the labeled substance, a tyramide compound to which the reactive group of the coumarin fluorescent dye is chemically bound, and hydrogen peroxide.

19. The staining kit according to any one of Claims 15 to 18, wherein the biological sample is also provided for fluorescence microscopic observation after being stained.
